# EUROPEAN PATENT APPLICATION

(11) **EP 4 549 019 A1**
(43) Date of publication of application: **07.05.2025**
(21) Application number: 24209469.6
(22) Date of filing: 29.10.2024
(51) Int. Cl.: B01L 3/00

(54) **CARTRIDGE FOR DETECTING TARGET ANALYTE**

(30) Priority: 03.11.2023 US 202318386775
(71) Applicant: Seegene, Inc., Seoul 05548 (KR)
(72) Inventor: CARISBROOKE, Huw John Joseph, Bentleigh East, 3165 (AU); STANTE, Anthony Joseph, Edithvale, 3196 (AU); KELLER, Cameron Douglas, Carlton, 3053 (AU); DIAS, Fernando Agostinho Peixoto, Moonee Ponds, 3039 (AU); MURRAY, Ross James, Olinda, 3788 (AU); KNOWLES, Stuart J., Oakleigh, 3166 (AU); GARDNER, Richard John Louis, Ascot Vale, 3032 (AU)
(74) Representative: Viering, Jentschura & Partner mbB Patent- und Rechtsanwälte

(57) **Abstract**

According to the disclosure, a cartridge for detecting a target analyte, comprising: a base comprising a first surface, a second surface opposite to the first surface, and side surfaces connecting between the first surface and the second surface; a chamber recessed from the first surface of the base; and an inflow channel recessed from the first surface of the base and connected to upper portion of the chamber. The chamber comprises an accommodation room for accommodating liquid, an inlet port connected the inflow channel, a guide groove for guiding liquid introduced from the inlet port downward to the accommodation room, and a barrier structure for preventing back flow of liquid from the guide groove toward the inlet port.

## Description

### BACKGROUND

### Field

The disclosure relates to a cartridge for detecting a target analyte.

### Description of Related Art

Nowadays people's interest in health increases and life expectancy extends. Thus, accurate analysis of pathogens and in vitro nucleic acid-based molecular diagnosis such as genetic analysis for a patient become significant, and the demand therefor is on the rise. Nucleic acid-based molecular diagnosis is performed by extracting nucleic acids from a sample and confirming whether a target nucleic acid is present in the extracted nucleic acids.

Polymerase chain reaction (PCR) is the most widely used nucleic acid amplification method, and the PCR process is performed by repeated cycling including denaturation of double-stranded DNA, annealing of oligonucleotide primers to the DNA templates and extension of primers by DNA polymerase (Mullis et al.; US Patent Nos. 4,683,195, 4,683,202 and 4,800,159; Saiki et al., (1985) Science 230, 1350-1354).

Real-time PCR using a fluorescent material is a method of detecting an increase in fluorescence intensity according to nucleic acid amplification during the PCR process. Real-time PCR enables multiplex detection by using different fluorescent dyes for each target; however, the technique requires expensive equipment and a lot of time for detection.

Meanwhile, recently, point of care testing which diagnoses patient's diseases quickly and correctly at any time and any place draws attention as a very significant technique of evidence-based precision health.

However, by the nature of the POC diagnostic device in which a simple and compact structure is critical, it is difficult to meet both fast processing and accurate detection.

### BRIEF SUMMARY

In the foregoing background, the disclosure provides a target analyte detection cartridge capable of safely dissolving a master mix dry reagent used to detect a target analyte.

In the foregoing background, the disclosure provides a target analyte detection cartridge capable of easily injecting a sample into the cartridge.

In the foregoing background, the disclosure provides a target analyte detection cartridge capable of preventing contamination of a channel when an extraction reaction of a sample solution occurs in a chamber accommodating magnetic beads.

In the foregoing background, the disclosure provides a target analyte detection cartridge capable of preventing backflow of liquid through an inflow channel in a chamber into which the liquid is introduced.

To achieve the foregoing objectives, an aspect of the disclosure A cartridge for detecting a target analyte, comprising: a base comprising a first surface, a second surface opposite to the first surface, and side surfaces connecting between the first surface and the second surface; a chamber recessed from the first surface of the base; and an inflow channel recessed from the first surface of the base and connected to upper portion of the chamber, wherein the chamber comprises an accommodation room for accommodating liquid, an inlet port connected the inflow channel, a guide groove for guiding liquid introduced from the inlet port downward to the accommodation room, and a barrier structure for preventing back flow of liquid from the guide groove toward the inlet port.

The accommodation room comprises a concave surface, an upper wall provided the inlet port, a lower wall, and side walls connecting between the upper wall and the lower wall, and wherein the guide groove comprises a portion extending downward in the direction of gravity from the side wall of the accommodation room.

The inlet port is located away from the side wall of the accommodation room, wherein the guide groove comprises a first portion extending along the upper wall of the accommodation room to adjacent the side wall and a second portion extending downward in the direction of gravity along the side wall of the accommodation room.

The guide groove comprises a portion which is deeper than the depth of the inlet port and shallower than the depth of the accommodation room.

The guide groove is configured to have a cross-sectional area within a range where a capillary phenomenon of the liquid introduced through the inflow channel could occur.

The barrier structure comprises a step that deepens in the direction from the inlet port toward the guide groove.

The barrier structure comprises a step that increases the cross-sectional area in the direction from the inlet port toward the guide groove.

The barrier structure comprises a barrier wall separating the inlet port and the guide groove.

The barrier wall is provided with a part of the upper wall of the accommodation room.

A distance between the inlet port and the guide groove is provided smaller than the maximum diameter of the droplet of the liquid introduced through the inflow channel.

The chamber further comprises a capillary groove provided on the lower wall of the accommodation room, and wherein the capillary groove is configured to have a cross-sectional area within a range where a capillary phenomenon of the liquid introduced through the inflow channel could occur.

The capillary groove extends to the side wall of the accommodation room where the guide groove is provided,

The guide groove is connected to the capillary groove.

The guide groove comprises a portion in which a cross-sectional area becomes smaller downward in the direction of gravity of the accommodation room.

The chamber further comprises a floor connection groove connecting the guide groove to a floor of the accommodation room.

The chamber further includes a blocking protrusion provided on the upper wall of the accommodation room, and wherein the blocking protrusion is provided in a direction opposite to the guide groove based on the inlet port.

The inlet port and the blocking protrusion are disposed apart from each other.

The cartridge may further comprise a cover provided on the one surface of the base to seal the inflow channel and the chamber.

The cartridge may further comprise a sample chamber into which a sample is introduced; a metering chamber connected to the sample chamber for quantifying the sample; a mixing chamber connected to the metering chamber and accommodating a magnetic bead; a liquid storage chamber connected to the mixing chamber and storing liquid; a detection chamber for detecting the target analyte; a waste chamber connected to the metering chamber; a plurality of liquid transport channels for transporting liquid of the liquid storage chamber to the mixing chamber; and a waste channel connecting the metering chamber and the waste chamber, wherein the chamber comprises the waste chamber, and the inflow channel comprises the waste channel.

The cartridge may further comprise a drain channel connecting the mixing chamber and the waste chamber, wherein the inflow channel comprises the drain channel.

According to an embodiment of the disclosure, it is possible to use the cartridge that is fast and accurate, yet small and lightweight.

Also, according to one embodiment, the master mix dry reagent used to detect the target analyte in the mixing chamber could be safely dissolved. Specifically, it is possible to sufficiently dissolve the master mix dry reagent while securing the enzyme stability.

Also, according to an embodiment, it is possible to increase the detection accuracy by preventing the channel from being contaminated when the extraction reaction of the sample solution occurs in the chamber accommodating the magnetic beads. Specifically, it is possible to increase the accuracy of the subsequent binding process and the elution process and purity of reactants by preventing contamination of the buffer and/or the pneumatic during the lysis process.

Also, according to one embodiment, it is possible to prevent backflow of liquid through the inflow channel in the chamber into which the liquid is introduced. Specifically, it is possible to prevent contamination of the mixing chamber due to backflow of the sample or solution drained into the waste chamber.

It should be appreciated that the effects of the disclosure are not limited thereto but may rather include all effects inferable from the configuration of the disclosure described in the detailed description or the claims of the disclosure.

### DESCRIPTION OF DRAWINGS

The above and other objects, features, and advantages of the disclosure will be more clearly understood from the following detailed description, taken in conjunction with the accompanying drawings, in which:
FIG. 1 is a plan view illustrating a cartridge according to an embodiment of the disclosure;
FIG. 2 is a perspective view illustrating a top view of a portion of the cartridge;
FIG. 3 is a perspective view illustrating a bottom view of FIG. 2;
FIG. 4 is a structural diagram illustrating a shuttle chamber arrangement according to an embodiment of the disclosure;
FIG. 5 is a structural diagram illustrating the shuttle chamber arrangement according to another embodiment of the disclosure;
FIG. 6 is a structural diagram illustrating the shuttle chamber arrangement according to another embodiment of the disclosure;
FIG. 7 is an enlarged view for explaining the shuttle chamber according to an embodiment of the disclosure;
FIG. 8 is a perspective view illustrating a front view of a sample chamber according to an embodiment of the disclosure;
FIG. 9 is a perspective view illustrating a rear of FIG. 8;
FIG. 10 is a vertical cross-sectional view for explaining the sample chamber;
FIG. 11 is an upper side view for explaining the sample chamber;
FIG. 12 is a plan view for explaining a waste chamber according to an embodiment of the disclosure;
FIG. 13 is a perspective view illustrating a bottom view of the waste chamber;
FIG. 14 is a partially enlarged view of FIG. 13;
FIG. 15 is a partially enlarged view illustrating various embodiments of a barrier structure; and
FIG. 16 is a partially enlarged view illustrating various embodiments of a lower portion of the waste chamber.

### DETAILED DESCRIPTION

Hereinafter, the disclosure will be explained with reference to embodiments and example drawings. The embodiments are for illustrative purposes only, and it should be apparent to a person having ordinary knowledge in the art that the scope of the disclosure is not limited to the embodiments.

In addition, in adding reference numerals to the components of each drawing, it should be noted that same reference numerals are assigned to same components as much as possible even though they are shown in different drawings. In addition, in describing the embodiments of the disclosure, when it is determined that a detailed description of a related well-known configuration or function interferences with the understanding of the embodiments of the disclosure, the detailed description thereof will be omitted.

In addition, in describing the components of the embodiments of the disclosure, terms such as first, second, A, B, (a), (b), (i), (ii), etc. may be used. These terms are only for distinguishing the components from other components, and the nature or order of the components is not limited by the terms. When a component is described as being "connected," "coupled" or "fastened" to other component, the component may be directly connected or fastened to the other component, but it will be understood that another component may be "connected," "coupled" or "fastened" between the components.

In the disclosure, the term "sample" may include a biological sample (e.g., cells, tissues and fluids from a biological source) and a non-biological sample (e.g., food, water and soil). Examples of the biological sample may include viruses, bacteria, tissues, cells, blood (e.g., whole blood, plasma and serum), lymph, bone marrow fluid, salvia, sputum, swab, aspiration, milk, urine, feces, ocular fluid, semen, brain extract, spinal fluid, joint fluid, thymus fluid, bronchoalveolar lavage fluid, ascites and amniotic fluid. Also, the sample may include natural nucleic acid molecules isolated from a biological source and synthetic nucleic acid molecules. According to an embodiment of the disclosure, the sample may include an additional substance such as water, deionized water, saline solution, pH buffer, acid solution or alkaline solution.

In the disclosure, the sample may include substances necessary for detecting a target analyte. For example, the sample may include an optical label. The optical label refers to a label that generates an optical signal depending on the presence of a target nucleic acid. The optical label may be a fluorescent label. The fluorescent label useful herein may include any molecule known in the art.

A target analyte refers to a substance that is the subject of analysis. The analysis may mean obtaining information on, for example, the presence, amount, concentration, sequence, activity or property of the analyte in the sample. The analyte may include various substances (e.g., biological substance and non-biological substance such as compounds). Specifically, the analyte may include a biological substance such as nucleic acid molecules (e.g., DNA and RNA), proteins, peptides, carbohydrates, lipids, amino acids, biological compounds, hormones, antibodies, antigens, metabolites or cells. According to an embodiment of the disclosure, the analyte may be nucleic acid molecules.

An apparatus for detecting a target analyte according to an embodiment of the disclosure may be an apparatus for detecting a nucleic acid and may detect a signal generated depending on the presence of the target nucleic acid. The apparatus for detecting a nucleic acid may amplify and detect a signal with nucleic acid amplification. Alternatively, the apparatus for detecting a nucleic acid may amplify and detect a signal without nucleic acid amplification. Preferably, the apparatus for detecting a nucleic acid detects a signal with nucleic acid amplification.

The nucleic acid reaction refers to sequential physical and chemical reactions which generate a signal depending on the presence of a nucleic acid of a specific sequence in the sample or the amount thereof. The nucleic acid reaction may include the binding of a nucleic acid of a specific sequence in a sample to other nucleic acids or substances, or replication, cleavage or decomposition of a nucleic acid of a specific sequence in the sample. The nucleic acid reaction may involve a nucleic acid amplification reaction. The nucleic acid amplification reaction may include amplification of a target nucleic acid. The nucleic acid amplification reaction may specifically amplify the target nucleic acid.

The nucleic acid reaction may a signal-generation reaction which can generate a signal depending on the presence/absence of a target nucleic acid in the sample or the amount thereof. The signal-generation reaction may be a technique of genetic analysis such as PCR, real-time PCR or microarray.

An apparatus for detecting a target analyte according to an embodiment of the disclosure may comprise a nucleic acid amplifier.

A nucleic acid amplifier refers to an apparatus for performing a nucleic acid amplification reaction which amplifies a nucleic acid having a specific nucleotide sequence. Examples of the method for amplifying a nucleic acid include polymerase chain reaction (PCR), ligase chain reaction (LCR), strand displacement amplification (SDA), transcription-mediated amplification, nucleic acid sequence-based amplification (NASBA), Q-beta Replicase, etc.

An apparatus for detecting a target analyte according to an embodiment of the disclosure may be an apparatus for performing a nucleic acid amplification reaction with temperature changes. For example, the nucleic acid amplifier may carry out a denaturing step, an annealing step and an extension (or elongation) step to amplify deoxyribonucleic acid (DNA) having a specific base sequence.

In the denaturing step, a sample and reagent solution containing double-stranded DNA templates is heated to a specific temperature, for example about 95°C, to separate double-stranded DNA into single-stranded DNA. In the annealing step, an oligonucleotide primer having a nucleotide sequence complementary to the nucleotide sequence of a nucleic acid to be amplified is provided, and the primer and the separated single-stranded DNA are cooled down to a specific temperature, for example 60°C, to promote the primer binding to the specific nucleotide sequence of the single-stranded DNA to form a partial DNA-primer complex. In the extension step, the solution is maintained at a specific temperature, for example 72°C, after the annealing step to form double-stranded DNA by DNA polymerase based on the primer of the partial DNA-primer complex.

The aforementioned three steps are repeated, for example 10 to 50 times, exponentially amplifying DNA having the specific nucleotide sequence. In some cases, the nucleic acid amplifier may perform the annealing step and extension step simultaneously. In this case, the nucleic acid amplifier may complete one cycle by performing two steps including a denaturing step and an annealing/extension step.

Particularly, an apparatus for detecting a target analyte according to an embodiment may be an apparatus for performing a nucleic acid amplification reaction with temperature changes and a reaction of generating an optical signal depending on the presence of a nucleic acid and detecting the generated optical signal.

Also, the apparatus for detecting a target analyte according to an embodiment may be a point of care (POC) diagnosis device, which is a miniaturized on-site diagnosis device.

The apparatus for detecting a target analyte may comprise a mounting module, a fluid transportation module, an extraction module, a thermo-control module, a sensing module, and a controller for controlling them.

The mounting module may include a manual type in which the user directly mounts the cartridge at a predetermined position and an automatic type in which the cartridge is received from the outside of the detection device, brought into the detection apparatus, and automatically placed in a predetermined position.

The fluid transportation module may provide power for transporting fluids including samples, reagents, or buffers. A reagent may be used as a meaning distinct from a buffer or may be used as a meaning including a buffer in some cases.

Fluid transport may be performed using syringe pumps when the cartridge is provided in a well type. Or fluid transport may be performed using micro pumps and micro valves inside or outside the micro-fluidic device when the cartridge is provided in a micro-fluidic type. The micro pumps and the micro valves often require additional driving force. Examples of drive mechanism for the micro pumps include check valves or peristaltic, rotary, centrifugal, ultrasonic, electro-hydrodynamic, electro-kinetic, phase transfer (requiring a change in temperature or pressure), electrowetting, magnetic, or hydrodynamic mechanism. And examples of drive mechanism for the micro valves include pneumatic, thermo-pneumatic, thermo-mechanical, piezoelectric, electrostatic, electromagnetic, electro-chemical, or capillary mechanism.

The extraction module may be used to extract a target analyte from a sample. For example, it can be used to extract nucleic acids from a sample. The extraction module may include a magnet used to manipulate magnetic beads to which nucleic acids are bound and a mixing means used to mix sample and reagent in the extraction process. For example, the mixing means may include an ultrasonic horn.

The thermo-control module may be used to control temperature during extraction or detection reaction of target analyte. For example, the thermo-control module may be used to control temperature during a nucleic acid extraction reaction or a nucleic acid amplification reaction. The temperature control module may include a heating element, a heat sink, a cooling fan, and a temperature sensor. For example, the heating element may include a Peltier element or a heating wire.

The sensing module may be used to detect a target analyte. Detection of the target analyte may use an optical method or an electrochemical method. For example, the sensing module may be an optical module.

The optical module may detect the target analyte using a method such as detection of change of fluorescence or color, or measurement of absorbance or reflectance. For example, the optical module can detect fluorescence emitted from a fluorescent material labeled on a target nucleic acid sequence.

And the optical module may include a light emitting unit supplying an appropriate optical stimulus to the sample accommodated in the sample holder and a detection unit detecting an optical signal generated from the sample in response thereto. The light emitting unit may include an LED or a laser, and the detection unit may include a charge coupled device (CCD), a complementary metal oxide semiconductor field effect transistor (CMOS), or a photodiode. The optical signal from the sample may be luminescence, phosphorescence, chemiluminescence, fluorescence, polarized fluorescence, or colored signal.

The electrochemical module may electrically sense the occurrence of a chemical reaction or a change in its degree according to the presence or absence of a target analyte in a sample or a change in its amount. For example, the electrochemical module may electrically sense a change in the target analyte in the sample by detecting a change in pH or resistance due to an increase in the target analyte or an electrochemical signal generated by binding of the target analyte and the active material.

And the electrochemical module may include an electrode unit and an electrical signal measuring unit. For example, the electrode unit may include a detection electrode made of gold (Au), cobalt (Co), platinum (Pt), silver (Ag), carbon nanotube, graphene, carbon, or an alloy containing any one or more of them. For example, the electrical signal measuring unit may be an anodic stripping voltammetry (ASV), a chronoamperometry (CA), a cyclic voltammetry, a square wave voltammetry (SWV), differential pulse voltammetry (DPV), or impedance.

Hereinafter, the directions used are set with respect to the drawings. For example, the cartridge is seated on the target analyte detection device in a standing state in which the upper portion shown in the figure is positioned up and may be mounted on the target analyte detection device while moving to the left direction of the figure. And when the cartridge is upright, "bottom", "lower", "below", or "downward" means the direction of gravity and "top", "upper", "above", or "upward" means the direction opposite to gravity.

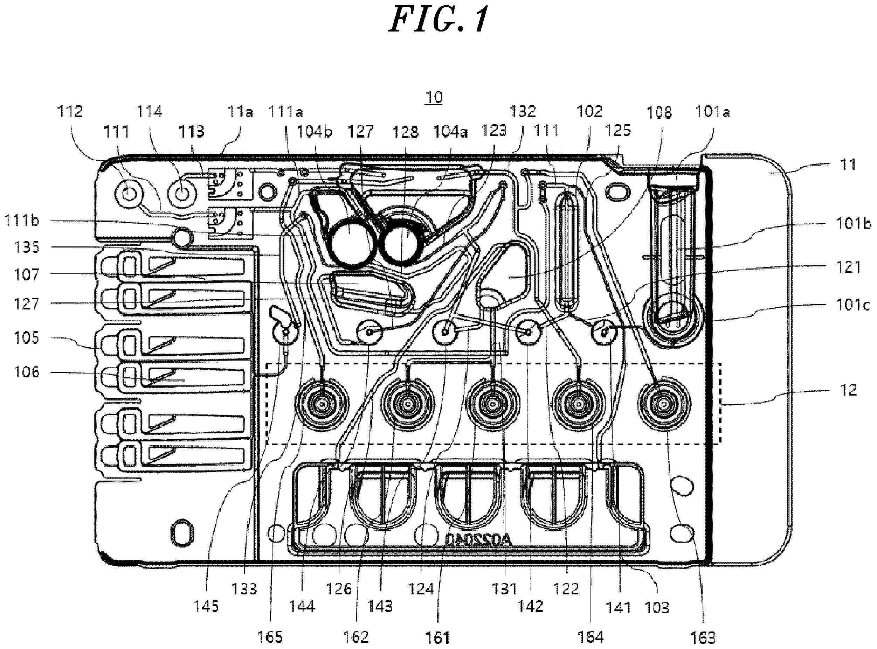

FIG. 1 is a plan view illustrating a cartridge 10 according to an embodiment of the disclosure.

Hereinafter, the first side (or front side) represents the plane shown in FIG. 1 and the second side (or back side) represents the back side of the plane shown in FIG. 1.

Referring to FIG. 1, a cartridge 10 of a target analyte detection device according to an embodiment of the disclosure may include a base 11 having a plurality of chambers and channels, a cover (transparent, not shown) sealing one or two opposite surfaces of the base 11, and a liquid storage chamber (not shown) fluidly coupled to the base 11 to provide a reagent or a buffer.

The liquid storage chamber may be provided as part of the base 11 integrally formed with the base 11. Alternatively, the liquid storage chamber may be provided as a separate member from the base 11 and may be attached to one surface of the base 11 through adhesion to be fluidly connected.

FIG. 1 shows a liquid storage chamber connecting portion 12 of a base 11 to which a liquid storage chamber is attached, and the liquid storage chamber is omitted from the illustration.

The base 11 may be provided to move in one direction (left direction in the FIG. 1) to be mounted with respect to the target analyte detection device (not shown). For example, a rack 11a is formed on an upper edge (or upper side) of the base 11 may be mechanically coupled with the pinion of the driving module provided in the target analyte detection device so that as the pinion rotates, the base 11 may move in one direction (left direction in the FIG. 1). The base 11 may slide in one direction by use of various mechanical structures, e.g., a piston structure, as well as the rack-pinion structure, and may not only slide but also pivot, rotation, or swing.

The base 11 may be a base plate provided in a plate shape. The base 11 may include a first surface (or front surface), a second surface (or rear surface), and side surfaces connecting the first surface and the second surface. The sides may include an upper side and a lower side.

A chamber, a channel, and a valve may be formed on at least one of the first surface and second surface of the base 11. The base 11 may include chambers for storing fluid, channels for connecting two or more chambers, and valves capable of opening and closing each channel.

The chamber and the channel of the base 11 may be formed by being concavely recessed in the first surface or second surface of the base 11 or may be formed through the first surface and the second surface of the base 11. And the chamber or channel may be sealed by a cover attached to the first surface or the second surface of the base 11.

The cover may be coupled or attached to one surface of the base 11 to seal any one or more of the chamber, channel, and valve provided on one surface of the base 11. Alternatively, if the chamber, the channel, and the valve are provided on the two opposite surfaces of the base 11, the cover may be coupled or attached to each of the two opposite surfaces of the base 11 to seal one or more of them. For example, the cover may include a first cover to seal the first surface of the base 11 and a second cover to seal the second surface of the base 11. The first cover and the second cover may be provided as a single cover or may include a plurality of cover elements provided so as not to overlap each other.

The cover includes a membrane coupled or attached to one surface of the base 11. As an example, the membrane used as the cover may be provided with a polymer membrane including a polycarbonate sheeted film or a metal membrane including an aluminum foil. The cover may be formed of other various materials and may also be provided as a plate, not as a membrane. In addition, the cover may be coupled to the base 11 through thermal compression, an adhesive, or mechanical coupling, etc.

Alternatively, both the base and the cover of the cartridge may be provided as a plate. And any one or more of the chamber, channel, and valve may be formed on a cover plate. Or any one or more of the chamber, channel, and valve may be formed on both the base plate and the cover plate.

The chamber may include a shape concavely recessed in a direction from the first surface to the second surface of the base 11. Specifically, the chamber may define a first surface opened from the first surface of the base 11, a second surface facing the first surface, and a side wall. The second surface of the chamber is located adjacent to the second surface of the base 11, and the side wall is defined as a side surface connecting the second surface of the chamber and the first surface of the base 11. Also, the first surface of the chamber may be a cover sealing the first surface of the base 11. Hereinafter, the first surface of the chamber may be expressed as a top surface, the second surface of the chamber may be expressed as a concave or a bottom surface, and the side wall of the chamber may be expressed as a side surface.

Alternatively, the chamber may include a shape concavely recessed in a direction from the second surface to the first surface of the base 11. Specifically, the chamber may define a first surface opened from the second surface of the base 11, a second surface facing the first surface, and a side wall. The second surface of the chamber is located adjacent to the first surface of the base 11, and the side wall is defined as a side surface connecting the second surface of the chamber and the second surface of the base 11. Also, the first surface of the chamber may be a cover sealing the second surface of the base 11.

Alternatively, the chamber may include a shape penetrating the first and second surfaces of the base 11. Specifically, the chamber may define a first surface opened from the first surface of the base 11, a second surface opened from the second surface of the base 11, and a side wall connecting the first and the second surfaces of the base 11. Also, the first surface of the chamber may be a first cover sealing the first surface of the base 11, and the second surface of the chamber may be a second cover sealing the second surface of the base 11.

Alternatively, the chamber may include both a shape concavely recessed in a direction from the first surface to the second surface of the bass 11 and a shape penetrating the first and second surfaces of the base 11. In the part where the chamber is concavely recessed in a direction from the first surface to the second surface of the base 11, the second surface of the chamber means a surface close to the second surface of the base 11. And in a part where the chamber is penetrating the second surface of the base 11, the second surface of the chamber means a cover sealing the second surface of the base 11.

The chamber may be connected to one or more channels. The channel connected to the chamber may be connected to the first surface, the second surface, or side wall of the chamber.

The chambers may include a sample chamber 101 into which samples are injected, a metering chamber 102 which meters a predetermined amount of samples, a waste chamber 103 which stores waste fluid, and a first mixing chamber 104a where a reaction between the sample and the reagent takes place, a second mixing chamber 104b for accommodating the dry reagent dissolved in a treatment solution (e.g., an eluted solution or eluate) sent from the first mixing chamber 104a, and a detection chamber 105 in which a sample amplification reaction (such as a PCR reaction, etc.) and a target analyte detection are processed.

The chamber may further include a shuttle chamber 107 connected to the second mixing chamber and used to dissolve the dry reagent, and an ante chamber 108 provided between the first mixing chamber 104a and the waste chamber 103.

The channel may include a first channel formed on the first surface of the base 11 and a second channel formed on the second surface of the base 11. Also, the first channel and the second channel may cross apart from each other. The channel may further include a through channel penetrating the base 11 to connect the first channel and the second channel.

The channels include a first pneumatic channel 111 and a second pneumatic channel 113 through which air pressure is transmitted, a sample channel 121 connecting between the sample chamber 101 and the metering chamber 102, and a metering channel 122 and the mixing channel 123 connecting between the metering chamber 102 and the first mixing chamber 104a, the first waste channel 125 connecting between the metering chamber 102 and the waste chamber 103, the mixing channel 123 and the second waste channel 126 connecting between the first mixing chamber 104a and the waste chamber 103, the mixing channel 123 and the mixing connection channel 127 connecting between the first mixing chamber 104a and the second mixing chamber 104b, a detection channel 135 connecting between the second mixing chamber 104b and the detection chamber 105, and buffer channels 131, 132, and 133.

The channel may further include an ante channel 124 connecting between the first mixing chamber 104a and the ante chamber 108 and a shuttle channel 128 connecting between the second mixing chamber 104b and the shuttle chamber 107.

In the disclosure, a meaning that a channel connects between chamber and chamber may be interpreted as the meaning including a case of direct connection and a case of indirect connection. In some cases, it may be interpreted as being limited to the case of direct connection.

In addition, in the disclosure, the distinguish of the channels may vary as needed. For example, one channel may be used as a meaning including another channel or may be used as a meaning distinguished from another channel in some cases. Also, the functions of the channels are not limited by the term of the channels. For example, the same channel may be designated by two or more terms on circumstances.

The valves may include a first valve 141 for opening and closing the sample channel 121, a second valve 142 for opening and closing the metering channel 122, a third valve 143 for opening and closing the mixing channel 123, and a fifth valve 145 for opening and closing the detection channel 135.

And the valve may further include a fourth valve 144 for opening and closing the mixing connection channel 127. In some cases, the third valve 143 may be used as a valve that opens and closes the ante channel 124.

According to an embodiment, the target analyte cartridge 10 may transfer fluid of the channel and the chamber using an air pressure. The base 11 may include a pneumatic channel communicating with the liquid channel. The pneumatic channel may be connected to a pneumatic means for applying positive or negative pressure. For example, the pneumatic means may include a pump.

The pneumatic channel may include a first pneumatic channel 111 and a second pneumatic channel 113. Further, the first and second pneumatic channels 111 and 113 may be selectively used as air injection channels or as air discharge channels. For example, the first and second pneumatic channels 111 and 113 may be connected to the pump through a means of switching direction, such as a solenoid valve.

The first pneumatic channel 111 may be used as an air injection channel and the second pneumatic channel 113 may be used as an air discharge channel, when the solenoid valve is in a first position. Conversely, the first pneumatic channel 111 may be used as an air discharge channel and the second pneumatic channel 113 may be used as an air injection channel, when the solenoid valve is in a second position. As such, the direction of fluid flow may be adjusted by switching the direction of pressure applied to the first and the second pneumatic channel 111, 113.

The cover may include pneumatic ports 112 and 114 for opening the pneumatic channels 111 and 113. The pneumatic ports may include a first pneumatic port 112 connected to the first pneumatic channel 111 and a second pneumatic port 114 connected to the second pneumatic channel 113.

The meaning of opening or closing the pneumatic ports 112 and 114 may include the meaning of operating or stopping the pneumatic means connected to the pneumatic ports 112 and 114. The meaning of applying pneumatic pressure to the pneumatic ports 112 and 114 may include the meaning of operating the pneumatic means connected to the pneumatic ports 112 and 114 to transfer the pneumatic pressure to the pneumatic channels 111 and 113.

In one aspect, the target analyte cartridge 10 may include a pre-processing region where the injected sample is preprocessed and a detection region which fluidly communicates with the second mixing chamber 104b, which is the last stage of the pre-processing region, and where detection of the target material occurs. For example, the pre-processing region may be provided on the right side of the cartridge 10, and the detection region may be provided on the left side of the cartridge 10. The sample injected from the right side of the cartridge 10 flows through the pre-processing region to the detection region and generally flows from the right side to the left side of the cartridge 10.

The pre-processing region and the detection region may be arranged in a horizontal direction. For example, the pre-processing region and the detection region may be arranged sequentially from right to left. In the detection region, a plurality of detection chambers may be arranged from top to bottom. For example, the detection region may include six detection chambers arranged from top to bottom.

In the pre-processing region, a chamber region, a valve region, and a buffer region may be arranged in a vertical direction. For example, the pre-processing region may include the chamber region, the valve region, and the buffer region arranged sequentially from top to bottom. The regions may partially overlap with each other.

In the chamber region, the sample chamber 101, the metering chamber 102, and the mixing chamber 104 may be arranged in a horizontal direction. For example, the chamber region may include the sample chamber 101, the metering chamber 102, and the mixing chamber 104 arranged sequentially from right to left.

In the valve region, a plurality of valves may be arranged in a horizontal direction. For example, the valve region may include the first valve 141, the second valve 142, the third valve 143, the fourth valve 144, and the fifth valve 145 arranged sequentially from right to left.

In the buffer region, a plurality of buffer chambers may be arranged in a horizontal direction. For example, the buffer region may include a first washing buffer chamber 163, a second washing buffer chamber 164, a lysis buffer chamber 161, a binding buffer chamber 162, and an elution buffer chamber 165 arranged sequentially from right to left. Additionally, the buffer chamber may include a blister chamber, and the buffer region may include a blister chamber region.

The waste region where the waste chamber 103 is provided may be placed below the buffer region. The waist region and the buffer region may partially overlap with each other.

In another aspect, the target analyte cartridge 10 may include a sample flow region including a chamber and a channel through which the sample flows and a liquid storage chamber region in which the liquid storage chamber is communicated. The target analyte cartridge 10 may further include a valve region where a valve for opening/closing the flow of the fluid between the sample flow region and the liquid storage chamber region is provided. For example, the sample flow region may be provided in an upper portion of the cartridge 10, the liquid storage chamber region may be provided in a lower portion of the cartridge 10, and the valve region may position between the sample flow region and the liquid storage chamber region 12.

FIG. 1 shows regions 161 to 165 to which each of the liquid storage chambers are attached and liquid transport channels 131, 132, and 133 connected to one or more liquid storage chambers. Although the liquid storage chambers are not disclosed in the drawings, the regions to which the liquid storage chamber is attached will be described instead the liquid storage chamber.

The liquid storage chamber may include a buffer chamber storing a buffer. A plurality of the buffer chambers may be disposed side by side in the liquid storage chamber region 12. For example, the first wash buffer chamber 163 and the second wash buffer chamber 164 in which wash buffer is stored, the lysis buffer chamber 161 in which lysis buffer is stored, a binding buffer chamber 162 in which a binding buffer is stored, and an elution buffer chamber 165 in which an elution buffer is stored may be disposed from the right side of FIG. 1.

Also, the liquid transport channel 131 to 133 connected to the liquid storage chamber may include a buffer channel. For example, the buffer channel may include the first buffer channel 131 connected to both the lysis buffer chamber 161 and the binding buffer chamber 162, the second buffer channel 132 connected to both the first washing buffer chamber 163 and the second washing buffer chamber 164, and the third buffer channel 133 connected to the elution buffer chamber 165.

The buffer chamber may be provided as a separate member from the base 11 and connected to the buffer channel of the base 11. In this case, the cover covering the first surface of the base 11 may form a through hole opening a portion where the buffer channel and the buffer chamber are fluidly connected.

The buffer chamber may be a blister chamber provided to transport the buffer while the membrane ruptures when pressure is applied. In addition, the blister chamber is fluidly connected to the buffer channel 131 to 133 of the base 11, and the buffer released under pressure may transferred to the target chamber along the buffer channel 131 to 133 of the base 11.

The detection device includes a pressurizing means for pressurizing the blister chamber to feed the buffer solution thereinside to the buffer channels 131 to 133 of the base 11. For example, the pressurizing means may include various means, such as a cam structure, a piston structure, a lever structure, or a magnet structure, etc.

Next, the connection relationship between the chamber, the channel, and the valve formed in the base 11 of the target analyte detection cartridge 10 is described below.

A fluid path may be formed between the first pneumatic channel 111 and the second pneumatic channel 113. Each chamber may communicate with one or more chambers through one or more channels. Each channel may be opened and closed by the valve. That is, chambers and channels may be connected between the first pneumatic channel 111 and the second pneumatic channel 113 to form a plurality of fluid paths, and the fluid flow may be adjusted by controlling the valve of the respective channels.

Hereinafter, the pneumatic channels 111 and 113 refer to channels in which a liquid fluid does not pass, and gas mainly flows. However, the pneumatic channels 111 and 113 do not exclude the case where fluid containing some liquid phase flows through the pneumatic channels 111 and 113.

An internal control bead, a protease K bead, and a magnetic bead may be provided in the fluid path. The internal control bead and the protease K bead may be prepared as dry reagents in a lyophilized state. Further, each bead may be provided in a space formed in the channel or may be provided inside the chamber. In addition, a master mix used for nucleic acid amplification may be provided inside the chamber, and the master mix may be prepared as a dry reagent in a liquid and/or lyophilized state.

The sample chamber 101 may include a hole through which the sample is injected and a space for receiving the injected sample. The sample may be injected by a pipette, or a separate container containing the sample may be inserted into the cartridge.

Also, the sample chamber 101 may be positioned in the right side of the cartridge 10, and the detection chamber 105 may be positioned in the left side of the cartridge 10. Thus, the sample may be transferred approximately from the right side to the left side of the cartridge 10.

According to an embodiment of the disclosure, the sample chamber 101 may include an injection hole 101a through which a sample is injected and storage space 101b and 101c into which the injected sample is stored. The storage space may include a sample delivery passage 101b connected to the injection hole 101a and delivering the sample in the direction of gravity, and a sample filter or sample filter mounting portion 101c provided between the outlet of the sample delivery passage 101b and the sample channel 121.

Referring to the drawing, the sample chamber 101 may be positioned at the upper edge of the base 11 so that the sample could be injected. Hereinafter, the edge refers to a side surface of the plate-shaped base 11 excluding the first surface and the second surface having a large area. When the base 11 is provided in a thin plate shape, an upper side surface of the base 11 on which the injection hole 101a is provided may expand in the thickness direction. In addition, the sample delivery passage 101b may extend from top to bottom to transfer the sample from the injection hole 101a to the sample channel 121.

Also, the injection hole 101a may be opened and closed by a closure. The closure is integrally formed with the base 11 to prevent loss.

Alternatively, unlike the drawing, the sample chamber 101 may be provided so that the sample is injected through the first surface of the base 11. In this case, the injection hole (not drawn) is provided as an opening formed on the first surface of the base 11, and the sample delivery passage is omitted so that the sample may be directly delivered from the injection hole to the sample filter 101c or the sample channel 121.

The sample chamber 101 may be connected to the sample channel 121 through an outlet of the sample chamber 101 located at the bottom. Also, the sample channel 121 may be opened and closed by the first valve 141.

The sample channel 121 may be connected to the sample chamber 101 on the second surface of the base 11, and the sample filter 101c may be mounted in a space formed on the second surface of the base 11. Also, the sample channel 121 may pass through the base 11 before or after the first valve 141 and be connected to the metering chamber 102 on the first surface of the base 11.

Although not shown in the drawings, a sample shutoff valve (not drawn) may be provided at an outlet of the sample chamber 101 or at a front end of the first valve 141 of the sample channel 121. For example, the sample shutoff valve may be a ball valve. In the initial state of the ball valve, a ball blocks the channel and, if the plunger pressurizes the ball by an operation signal, the ball moves off to open the blocked channel. The ball of the sample shutoff valve may be provided not to return to the initial position after moving off. For example, the ball may move off and drop downward by gravity.

The sample channel 121 may be opened and closed by the first valve 141. The first valve 141 may be provided in a closed state in an initial state and converted to an open state by an operation signal. This is because if the sample is injected while the first valve 141 is open, bubbles may be generated in the process of leaking the sample along the sample channel 121 and the accuracy of the subsequent metering step may be reduced.

The sample channel 121 is connected to the metering chamber 102. For example, the sample channel 121 is branched in the middle, so that one branch channel is connected to the metering chamber 102 and the other branch channel is connected to the metering channel 122. The metering channel 122 is connected to the first mixing chamber 104a.

Meanwhile, a portion of the channel connected from the branch point located at the rear end of the first valve 141 to the metering chamber 102 may be selectively termed as the sample channel 121 or the metering channel 122 as needed.

The metering chamber 102 may be used to meter a required quantity (or predetermined amount) of sample. Hereinafter, the quantity of sample means a predetermined amount of sample. The metering chamber 102 may be positioned close to the sample chamber 101 and may, for example, be positioned to the left of the sample chamber 101. Also, the metering chamber 102 may be elongated in a vertical direction (or a direction of gravity). Also, the metering chamber 102 may include a portion located higher than the sample channel 121. Furthermore, the lower part of the metering chamber 102 to which the sample channel 121 or the metering channel 122 is connected may be located higher than the lower part of the sample chamber 101 to which the sample channel 121 is connected.

The metering chamber 102 may be connected to the channels both the top and bottom. Air bubbles present in the sample move to the channel connected to the top of the metering chamber 102, so that the metering chamber 102 may be filled with the sample therein. If air bubbles exist in the metering chamber 102, a quantity of sample may not be metered, and detection accuracy may decrease.

The channel connected to the upper portion of the metering chamber 102 may be connected to the first pneumatic channel 111 and the first waste channel 125, respectively. More specifically, the first pneumatic channel 111, the first waste channel 125, and the metering chamber 102 converge at a junction (or convergence point). One channel connected to the first pneumatic port 112 at the junction may be termed as the first pneumatic channel 111, another channel connected to the metering chamber 102 at the junction may be termed as an upper metering channel, and the other channel connected to the waste chamber 103 at the junction may be termed as a waste channel 125. In this case, the upper metering channel may be a part of the metering chamber 102. That is, the upper part of the metering chamber 102 may be directly connected to the junction of the first pneumatic channel 111 and the first waste channel 125.

Meanwhile, in the disclosure, a convergence point and a divergence point (or branch point) may be expressed or understood as a junction without being distinguished from each other. For example, a point where three channels meet may be expressed as a divergence point where one channel diverges into two or more channels, or a convergence point where three channels converge, as needed.

The first pneumatic channel 111 may be a channel through which air flows without flowing liquid, and the first waste channel 125 may mean a channel through which liquid flows from the metering chamber 102 to the waste chamber 103. there is. However, the case where some liquid fluid flows in the first pneumatic channel 111 is not excluded.

The inlet of the first pneumatic channel 111 may be connected to the pneumatic means through the first pneumatic port 112. For example, a first pneumatic port 112 and a second pneumatic port 114 may be provided on the upper left side of the base 11. The first pneumatic port 112 and the second pneumatic port 114 located above the detection chamber 105 may be connected to a pneumatic means (e.g., a pump) in the detection device while the cartridge is mounted in the detection device.

Also, the first pneumatic channel 111 may be connected to the first waste channel 125 at the convergence point connected to the metering chamber 102. That is, the channel connected to the metering chamber 102 (including a part of the metering chamber 102), the first pneumatic channel 111, and the first waste channel 125 may meet each other at one point.

The first waste channel 125 may extend downward and be connected to the waste chamber 103. Or the first waste channel 125 may extend in the direction of gravity and be connected to the waste chamber 103. In addition, the first waste channel 125 includes a portion where the cross-sectional area of the channel increases in the direction of the waste chamber 103, so that reverse flow from the waste chamber 103 toward the metering chamber 102 may be prevented.

The waste chamber 103 may be positioned below the liquid storage chamber 12 and may extend long in the horizontal direction to secure a volume. The first waste channel 125 may be connected to one side of the upper portion of the waste chamber 103, and the second waste channel 126 may be connected to the other side of the upper portion of the waste chamber 103. The waste chamber 103 may be connected to the first pneumatic channel 111 through the first waste channel 125 and connected to the second pneumatic channel 113 through the second waste channel 126. The waste chamber 103 may be ventilated through the second pneumatic port 114 by passing through the second waste channel 126 and the second pneumatic channel 113. Specifically, the first waste channel 125 may be connected to the right side of the upper portion of the waste chamber 103, and the second waste channel 126 may be connected to the left side of the upper portion of the waste chamber 103.

The second waste channel 126 may be connected to the mixing channel 123 by passing through the ante chamber 108 and the ante channel 124. Also, the mixing channel 123 may be connected to the first mixing chamber 104a, and the first mixing chamber 104a may be connected to the second pneumatic channel 113. That is, the second waste channel 126 may be connected to the second pneumatic channel 113. Alternatively, the second waste channel 126 may be directly connected to the mixing channel 123 when the ante chamber 108 is not provided.

Also, the second waste channel 126 may be a drain channel or a part of the drain channel. The drain channel means a channel used to drain the waste fluid of the first mixing chamber 104a to the waste chamber 103.

The metering chamber 102 may be connected to the first mixing chamber 104a through the metering channel 122 and the mixing channel 123. And a second valve 142 may be provided for opening and closing the metering channel 122. For convenience, a channel passing through the second valve 142 is referred to as the metering channel 122, and a channel between a branch point located at the rear end of the second valve 142 and the first mixing chamber 104a is referred to as the mixing channel 123. there is. However, if necessary, the mixing channel 123 may be interpreted as including the metering channel 122, and it is also possible to refer to the metering channel 122 and the mixing channel 123 interchangeably in some sections.

The first mixing chamber 104a may be connected to the waste chamber 103 through the mixing channel 123, the ante channel 124, and the second waste channel 126. And a third valve 143 may be provided for opening and closing the ante channel 124. For convenience, a channel passing through the third valve 143 is referred to as an ante channel 124, and a channel between a branch point located at the rear end of the third valve 143 and the first mixing chamber 104a is referred to as the mixing channel 123. However, if necessary, the mixing channel 123 may be interpreted as including the ante channel 124.

Alternatively, the first mixing chamber 104a may be connected to the waste chamber 103 through the mixing channel 123 and the second waste channel 126 when the ante chamber 108 is not provided. And a third valve 143 may be provided for opening and closing the mixing channel 123. For convenience, a channel passing through the third valve 143 is referred to as the mixing channel 123, and a channel between a branch point located at the rear end of the third valve 143 and the waste chamber 103 is referred to as the second waste channel 126. However, if necessary, the mixing channel 123 may be interpreted as including the second waste channel 123.

Also, the first mixing chamber 104a may accommodate an internal control bead (IC bead), proteinase K bead, and magnetic beads therein.

According to an embodiment of the disclosure, the magnetic incorporate particles of iron oxides, such as magnetite (Fe₃O₄), which give them superparamagnetic properties. Unlike more common ferromagnets, superparamagnetic beads exhibit magnetic behavior only in the presence of an external magnetic field. This paramagnetic property, which depends on the particles in the beads, allows the beads to be separated in suspension, along with anything they are bound to. Since they don't attract each other outside of a magnetic field, they can be used without any concern about unwanted clumping. For example, the magnetic beads may specifically bind or dissociate nucleic acids depending on pH.

The magnetic bead may include a magnetic core, a polymer shell surrounding the core, and a reactor binding to cell or nucleic acid. For example, the magnetic bead may be a core-shell structure including a core of iron oxides and a silica shell surrounding the core. And the magnetic bead may further include a polymer shell to prevent toxic exposure of the core.

The first mixing chamber 104a may include an electromagnetic field receiving area to which an electromagnetic field is transmitted by a magnetic means (or an electromagnetic field generating part) installed in the target analyte detection device. The electromagnetic field receiving area may be provided in a reaction space of the first mixing chamber 104a. The magnetic beads in the first mixing chamber 104a may be provided to be movable in response to the electromagnetic field of a magnetic means.

The magnetic means may be located close to one surface (e.g., the first surface of the base 11) of the cartridge 10 mounted on the target analyte detection device. The magnetic means generates the electromagnetic field to capture the magnetic beads in the first mixing chamber 104a).

The magnetic means may be turned on/off by an electric signal. Alternatively, the magnetic means may maintain an operating state during the processing operation of the cartridge 10 and may adjust the strength of the electromagnetic field acting on the first mixing chamber 104a while changing its relative position with respect to the first mixing chamber 104a. For example, when the magnetic means is switched to an operating state while being located close to the first surface of the first mixing chamber 104a or when the magnetic means is located close to the first surface of the first mixing chamber 104a in an operating state, the magnetic beads may be fixed to the first surface of the first mixing chamber 104a.

Furthermore, the magnetic means may move position along the first surface of the first mixing chamber 104a in the operating state. As the magnetic means moves, the captured magnetic beads move together along the magnetic means, and extra magnetic beads may be additionally captured by the magnetic means.

The protease K beads may be accommodated inside the first mixing chamber 104a or the second mixing chamber 104b. Preferably, the protease K beads may be accommodated inside the first mixing chamber 104a.

In addition, the first mixing chamber 104a and/or the second mixing chamber 104b may include an ultrasonic receiving area to which an ultrasonic wave is transmitted by an ultrasonic means (or ultrasonic generator) installed in the target analyte detection device. The ultrasonic receiving area may be provided in a reaction space of the first mixing chamber 104a and/or the second mixing chamber 104b.

The ultrasonic means may be located close to one surface (e.g., the first surface of the base 11) of the cartridge 10 mounted on the target analyte detection device. The ultrasonic means generate ultrasonic waves while vibrating to promote a mixing reaction or dissolution reaction in the first mixing chamber 104a or the second mixing chamber 104b. The ultrasonic means may promote mixing of the sample and reagents or reagents and buffers. Or the ultrasonic means may promote the dissolution of dry reagents. For example, the ultrasonic means may include an ultrasonic waves horn.

In addition, the first mixing chamber 104a, the second mixing chamber 104b, and/or the detection chamber 105 may include a heat receiving area to which heat is transferred by a heating means (or heat generating unit) installed in the target analyte detection device. The heat receiving area may be provided in a reaction space of the first mixing chamber 104a, the second mixing chamber 104b, and/or the detection chamber 105.

The heating means may be located close to one surface (e.g., the first surface of the base 11) of the cartridge 10 mounted on the target analyte detection device. The heating means generates heat by a heating element to increase the temperature inside the first mixing chamber 104a, the second mixing chamber 104b, or the detection chamber 105. The heating means may cause or promote a mixing reaction, dissolution reaction, amplification reaction, or chemical reaction. For example, the heating element may include a Peltier element or a thermal resistance element.

Alternatively, the first mixing chamber 104a and/or the second mixing chamber 104b may include a heating element to generate heat by itself.

Meanwhile, the magnetic means, the ultrasonic means, and the heating means may be provided to be relatively movable with each other. For example, the detection device may include a wheel structure capable of rotating or revolution. The wheel structure may include a wheel base, a rotation axis of the wheel base, and a motor that rotates the rotation axis of the wheel base. The magnetic means, the ultrasonic means, and the heating means may be installed apart from each other in the wheel structure. While the wheel structure rotates, the magnetic means, the ultrasonic means, or the heating means may be positioned adjacent to the first mixing chamber 104a and/or the second mixing chamber 104b, respectively.

The magnetic means may be referred to as a magnetic unit, the ultrasonic means may be referred to as an ultrasonic unit, and the heating means may be referred to as a heating unit.

In addition, the wheel structure is provided in a size capable of covering both the first mixing chamber 104a and the second mixing chamber 104b, so that each of the chambers 104a and 104b may be operated by single ultrasonic means or single heating means.

The first mixing chamber 104a may include a first surface, a second surface, and a side wall. The first surface of the first mixing chamber 104a may be provided with a membrane (e.g., a sheet film) sealing the penetrating portion of the first surface of the base 11. The second surface of the first mixing chamber 104a may be provided as a surface located close to the second surface of the base 11 when it is provided concavely toward the second surface of the base 11, and may be provided with a membrane (e.g., sheet film) sealing the penetrating portion of the second surface of the base 11 when the second surface of the base 11 is penetrated. The side wall of the first mixing chamber 104a may be provided as a wall connecting the first surface and the second surface thereof.

In addition, the first mixing chamber 104a may be divided into a lower space provided in the first area and an upper space provided in the second area. The first area and the second area of the first mixing chamber 104a may be spatially separated while being connected to each other.

The first area (or upper space) may be provided as an area (or space) where fluid flows from the liquid storage chamber or communicates with the pneumatic channel. The second area (or lower space) may be provided as an area (or space) where accommodates the magnetic beads and occurs some reactions.

The first area of the first mixing chamber 104a may be provided concavely toward the second surface of the base 11, and the second surface of the first area may be a concave surface (or bottom surface) located close to the second surface of the base 11. The second area of the first mixing chamber 104a may be provided to penetrate the second surface of the base 11, and the second surface of the second area may be a cover sealing the second surface of the base 11. The cover may be provided with a membrane (e.g., sheet film). Specifically, the cover may be provided with a polycarbonate sheeted film.

The membrane constituting the second surface of the lower space of the first mixing chamber 104a may use a material having excellent ultrasonic conductivity. Ultrasonic waves generated from the ultrasonic means may be transmitted to the lower space through the membrane, and loss may be small. In addition, the membrane constituting the second surface of the lower space may use a material having excellent electromagnetic field permeability. In addition, the membrane constituting the second surface of the lower space may use a material having excellent thermal conductivity.

The first area of the first mixing chamber 104a may be provided with a hole through which liquid is introduced and a hole through which the second pneumatic channel 113 is connected. The fluid flowing into the first mixing chamber 104a may include a buffer.

The second area of the first mixing chamber 104a may communicate with the mixing channel 123. The second area of the first mixing chamber 104a may be introduced a sample, a binding buffer, and a lysis buffer through the mixing channel 123. The second area may be drained a waste fluid and transferred the solution in which the elution reaction has occurred to the second mixing chamber 104b. Specifically, the sample metered in the metering chamber 102 may pass through the metering channel 122 and the mixing channel 123 and flow into the lower space of the first mixing chamber 104a. The buffers of the lysis buffer chamber 161 and the binding buffer chamber 162 may pass through the first buffer channel 131 and the mixing channel 123 and flow into the lower space of the first mixing chamber 104a. Also, the waste fluid of the first mixing chamber 104a may be drained into the waste chamber 103 through the mixing channel 123 and the second waste channel 126.

The lower space of the first mixing chamber 104a may be provided deeper than the upper space. The lower space may be a space where a sample and a buffer or a sample and a reagent are mixed, and a reaction occurs.

The first mixing chamber 104a may include a structure capable of preventing the mixed solution from splashing into the upper space while mixing occurs in the lower space. For example, a blocking wall may be formed in a portion of the first mixing chamber 104a except for a channel through which fluid may move between the upper space and the lower space.

In addition, the lower space of the first mixing chamber 104a may be connected to the lysis buffer chamber 161 and the binding buffer chamber 162 through the first buffer channel 131 and may be connected to the first wash buffer chamber 163 and the second wash buffer chamber 164 through the second buffer channel 132. The first buffer channel 131 may be branched and connected to the lysis buffer chamber 161 and the binding buffer chamber 162, respectively. The second buffer channel 132 may be branched and connected to the first washing buffer chamber 163 and the second wash buffer chamber 164, respectively.

The first buffer channel 131 may pass through the ante chamber 108 and the ante channel 124 and be connected to the mixing channel 123. Also, the mixing channel 123 may be connected to the first mixing chamber 104a, and the first mixing chamber 104a may be connected to the second pneumatic channel 113. That is, the first buffer channel 131 may be connected to the second pneumatic channel 113.

Alternatively, the first buffer channel 131 may be directly connected to the mixing channel 123 when the ante chamber 108 is not provided. Also, the first buffer channel 131 may have one end connected to the mixing channel 123 and the other end may branch into a channel connected to the lysis buffer chamber 161 and a channel connected to the binding buffer chamber 162. For convenience, a channel passing through the third valve 143 is referred to as a mixing channel 123, and a channel between the third valve 143 and the lysis buffer chamber 161 and a channel between the third valve 143 and the binding buffer chamber 162 are referred to as a first buffer channel 131. However, if necessary, the mixing channel 123 may be interpreted as including the first buffer channel 131, and it is also possible to refer to the mixing channel 123 and the first buffer channel 131 interchangeably in some sections.

According to an embodiment, the first mixing chamber 104a may be connected to the ante chamber 108 through the mixing channel 123 and the ante channel 124. The ante chamber 108 may be connected to the waste chamber 103 through the second waste channel 126 and be connected to the lysis buffer chamber 161 and the binding buffer chamber 162 through the first buffer channel 131. The ante chamber 108 will be described in detail below.

In addition, the upper space of the first mixing chamber 104a may be connected to the first and second wash buffer chambers 163 and 164 through the second buffer channel 132. And the upper space may be connected to the elution buffer chamber 165 through the third buffer channel 133. For example, the right side of the upper space of the first mixing chamber 104a may be connected to the second buffer channel 132, and the left side of the upper space may be connected to the third buffer channel 133.

The second mixing chamber 104b may accommodate reagents used in the nucleic acid amplification reaction therein. The reagent accommodated in the second mixing chamber 104b may include a liquid reagent and/or a dry reagent. For example, the dry master mix reagent used in the nucleic acid amplification reaction may be prepared in a lyophilized state and accommodated in the second mixing chamber 104b.

The second mixing chamber 104b may have one side connected to the first mixing chamber 104a and the other side connected to the detection chamber 105.

In addition, the second mixing chamber 104b may be divided into a lower space provided in the first area and an upper space provided in the second area. The first area and the second area of the second mixing chamber 104b may be spatially separated while being connected to each other.

The first area (or upper space) may be provided as an area (or space) where communicates with the pneumatic channel. The second area (or lower space) may be provided as an area (or space) for accommodating the dried reagent and dissolving it.

The first area of the second mixing chamber 104b may be provided concavely toward the second surface of the base 11, and the second surface of the first area may be a concave surface (or bottom surface) located close to the second surface of the base 11. The second area of the second mixing chamber 104b may be provided to penetrate the second surface of the base 11, and the second surface of the second area may be a cover sealing the second surface of the base 11. The cover may be provided with a membrane (e.g., sheet film). Specifically, the cover may be provided with a polycarbonate sheeted film.

The membrane constituting the second surface of the lower space of the first mixing chamber 104a may use a material having excellent ultrasonic conductivity. Ultrasonic waves generated from the ultrasonic means may be transmitted to the lower space through the membrane, and loss may be small. In addition, the membrane constituting the second surface of the lower space may use a material having excellent electromagnetic field permeability. In addition, the membrane constituting the second surface of the lower space may use a material having excellent thermal conductivity.

The upper space of the second mixing chamber 104b may be connected to the first pneumatic channel 111. For example, one end of the first pneumatic channel 111 may be communicates to the first pneumatic port 112. The first pneumatic channel 111 may be branched in the middle so that the first branch channel 111a is connected to the second mixing chamber 104b and the second branch channel 111b is connected to the metering chamber 102 and the first waste channel 125.

The lower space of the second mixing chamber 104b may be provided deeper than the upper space and accommodate the dry reagent. The lower space may be a space in which the dry reagent is dissolved in the eluate delivered from the first mixing chamber 104a. Also, the lower space may be connected to the detection chamber 105 through the detection channel 135.

The cartridge according to one embodiment may further include a shuttle chamber 107 positioned between the first mixing chamber 104a and the second mixing chamber 104b. The shuttle chamber 107 will be described in detail below.

The detection channel 135 may have one end connected to the second mixing chamber 104b and other end connected to the plurality of detection chambers 105. The detection channel 135 may be opened and closed by the fifth valve 145. Also, the detection channel 135 may branch into a plurality of branch channels corresponding to the plurality of detection chambers 105 at the fifth valve 145 or at a rear end of the fifth valve 145. For example, the detection channel 135 may be branched into six branch channels each connected to the six detection chambers 105.

The fifth valve 145 may further include an air collecting space. Air bubbles generated during the mixing process in the second mixing chamber 104b may move to the air collection space provided above the fifth valve 145 so that the liquid free from air bubbles may be moved through the detection channel 133.

The plurality of detection chambers 105 may be positioned side by side along the left edge of the base 11 in the vertical direction. For example, the plurality of detection chambers 105 may be provided close to an end portion where the cartridge 10 is inserted into the detection device. The target analyte detection device may be provided with a detection module capable of inspecting whether the target analyte is present in the detection chambers 105 provided in the inserted cartridge 10. For example, the detection module may include an LED that emits excitation light to the detection chamber 105 and a photodiode that receives reflected light reflected from the detection chamber 105. A plurality of LEDs and photodiodes may be provided to correspond to each of the plurality of detection chambers 105.

The detection chamber 105 may include a detection reagent therein. The detection reagent may include a lyophilized oligonucleotide primer. If the detection solution flows into the detection chamber 105 through the detection channel 133, an amplification reaction may occur as the detection reagent is dissolved.

The target analyte detection device may include a detection chamber heating means for transferring heat to the detection chamber 105. If heat is transferred from the detection chamber heating means, the temperature of the detection chamber 105 is increased to and maintained at a certain temperature so that an amplification reaction may occur inside the detection chamber 105.

In addition, the front (first surface) and rear surface (second surface) of the detection chamber 105 may be provided to pass through the base 11. The penetrated front and back surfaces may be sealed with a membrane (e.g., sheet film). A membrane constituting the detection chamber 105 may use a material having excellent light transmittance and thermal conductivity.

Each detection chamber 105 may be connected to the detection buffer chamber 106. The detection buffer chamber 106 may be a space for receiving the detection solution overflowing the detection chamber 105, a space for storing the air of the detection channel 135 and the detection chamber 105, or a space for storing the evaporated gas in the detection chamber 105.

The six detection buffer chambers 106 may all have the same volume. As the air is pushed out while the detection solution moves through the detection channel 135 and the detection chamber 105 and is stored in each buffer chamber 106, the six detection buffer chambers 106 all may provide the same reaction force. Accordingly, the same amount of detection solution may be provided to each detection chamber 105.

In addition, the front (first surface) and rear surface (second surface) of the detection buffer chamber 106 may be provided to pass through the base 11. The penetrated front and back surfaces may be sealed with a membrane (e.g., sheet film). A membrane constituting the detection buffer chamber 106 may use a material capable of elastic deformation. When the detection solution is filled in the detection buffer chamber 106, an excess amount of the detection solution may be additionally retained while the membrane is elastically deformed.

A process for detecting a target analyte using the cartridge 10 according to an embodiment is described below.

A cartridge loading unit of the target analyte detection device may wait for a user input to execute the protocol.

### [Sample loading and cartridge mounting]

The user may load the sample into the injection hole 101a of the sample chamber 101. For example, after opening the closure and injecting the sample into the injection hole 101a through a pipette, the user may close and seal the closure. The sample injected through the injection hole 101a may be accommodated in the storage space (the sample delivery passage 101b and the sample filter mounting portion 101c) while moving downward by gravity.

The user may seat or insert and mount the cartridge 10 into the insertion portion of the target analyte detection device and then input an operation signal for starting the detection process through the input unit of the detection device. Alternatively, after recognizing the mounting or insertion of the cartridge 10 in the insertion portion, the detection device may perform the protocol after a predetermined time has passed without the user's operation signal.

The cartridge loading unit performs the following protocol.

If the mounting of the cartridge 10 is recognized, a mounting member of the cartridge loading unit where the cartridge 10 is mounted clamps the cartridge 10. The mounting member moves the cartridge 10 to the operation position. The operating position of the cartridge 10 may be the same as the detection position. For example, if the cartridge 10 is moved to the operation position, the detection chamber 105 may be positioned below the detection module.

In the initial state before the process of the cartridge 10 is started, all the valves 141 to 145 and the pneumatic channels 111 and 113 may be in the open state. However, the sample shutoff valve (not drawn) provided at the outlet or the rear end of the outlet of the sample chamber 101 may be in the closed state in the initial state. The pressurizing module that applies pressure to the blister chamber may also be in the initial state (open state).

### [Sample metering process]

Thereafter, the detection device may perform a metering process of metering a predetermined amount of sample in the sample chamber 101.

The valve controller may open the first valve 141 and the third valve 143 while closing the remaining valves. The sample stored in the sample chamber 101 may flow to the sample channel 121 while the first valve 141 provided at the rear end of the outlet of the sample chamber 101 is opened.

The pneumatic controller closes the first pneumatic port 112 and opens the second pneumatic port 114 and then may apply negative pressure through the second pneumatic port 114. The applied negative pressure may be sequentially transferred to the second pneumatic channel 113, the first mixing chamber 104a, the mixing channel 123, the third valve 143, the second waste channel 126, the waste chamber 103, the first waste channel 125, the metering chamber 102, the sample channel 121, and the sample chamber 101.

Then, the sample in the sample chamber 101 fills the metering chamber 102 and overflows the metering chamber 102. And then, the overflowed sample moves until filling a portion of the first waste channel 125 or may reach through the first waste channel 125 to the waste chamber 103.

Next, the valve controller may switch the first valve 141 to the closed state. The pneumatic controller opens the first pneumatic port 112 and the second pneumatic port 114 and then may apply positive pressure to the first pneumatic port 112 or negative pressure to the second pneumatic port 114.

According to one embodiment, the positive pressure applied through the first pneumatic port 112 may be in turn transferred to the first pneumatic channel 111, the first waste channel 125, the waste chamber 103, the second waste channel 126, the third valve 143, the mixing channel 123, the first mixing chamber 104a, and the second pneumatic channel 113. Alternatively, when negative pressure is applied through the second pneumatic port 114, the pneumatic pressure may be transmitted through the opposite path above.

In this process, the sample remaining in the first waste channel 125 may be discarded to the waste chamber 103. As the sample overflowing the metering chamber 102 is discarded into the waste chamber 103, the volume of the sample filling the metering chamber 102 may be metered. The process of blowing air to remove the overflowed sample overflowing the metering chamber 102 may be referred to as an air knife process.

Then, the process of transferring the metered sample to the first mixing chamber 104a may proceed.

The valve controller may switch the second valve 142 to the open state and may switch the third valve 143 to the closed state. The pneumatic controller may apply positive pressure to the first pneumatic port 112 or negative pressure to the second pneumatic port 114.

According to one embodiment, the applied positive pressure through the first pneumatic port 112 may be sequentially transferred to the first pneumatic channel 111, the metering chamber 102, the metering channel 122, the second valve 142, the mixing channel 123, and the first mixing chamber 104a. In this process, the sample filling the metering chamber 102 may be transferred to the first mixing chamber 104a through the metering channel 122, the second valve 142, and the mixing channel 123. Alternatively, when negative pressure is applied through the second pneumatic port 114, the pneumatic pressure may be transmitted through the opposite path above.

The internal control bead positioned in the first mixing chamber 104a may be dissolved in the sample delivered to the first mixing chamber 104a.

### [Lysis process]

Thereafter, the lysis buffer may be transferred to the first mixing chamber 104a, and the lysis process may be performed in the first mixing chamber 104a.

The valve controller may switch the second valve 142 to the closed state and switch the third valve 143 to the open state. The pneumatic controller may close the first pneumatic port 112 and apply negative pressure through the second pneumatic port 114. The applied negative pressure may be sequentially transferred to the second pneumatic channel 113, the first mixing chamber 104a, the mixing channel 123, the third valve 143, and the first buffer channel 131.

The blister pressurizing means may simultaneously pressurize the lysis buffer chamber 161 to supply the lysis buffer. In this process, the lysis buffer of the lysis buffer chamber 161 is transferred to the first mixing chamber 104a through the first buffer channel 131, the third valve 143, and the mixing channel 123. The protease K bead positioned in the first mixing chamber 104a may be dissolved in the lysis buffer.

The ultrasonic means may move to a position corresponding to the first mixing chamber 104a and generate ultrasonic waves in the first mixing chamber 104a to mix the sample and the lysis buffer.

Then, the valve controller may switch the third valve 143 to a closed state. The pneumatic controller may close the first pneumatic port 112 and apply positive pressure through the second pneumatic port 114. Since the valves are all closed, the positive pressure may increase the pressure in the first mixing chamber 104a. The heating means may move to a position corresponding to the first mixing chamber 104a and apply heat to the first mixing chamber 104a to increase the temperature within the chamber.

As such, the incubating process may be performed for a predetermined time while the pressure and temperature of the first mixing chamber 104a are increased.

### [Binding process]

Thereafter, the binding buffer is transferred to the first mixing chamber 104a, and the binding process may be performed in the first mixing chamber 104a.

The valve control unit may switch the third valve 143 to an open state. The pneumatic controller may apply negative pressure through the second pneumatic port 114. The applied negative pressure may be sequentially transferred to the second pneumatic channel 113, the first mixing chamber 104a, the mixing channel 123, the third valve 143, and the first buffer channel 131.

The blister pressurizing means may simultaneously pressurize the binding buffer chamber 162 to supply the binding buffer. In this process, the binding buffer of the binding buffer chamber 162 is transferred to the first mixing chamber 104a through the first buffer channel 131, the third valve 143, and the mixing channel 123.

Then, the valve controller may switch the third valve 143 to the closed state and switch the second valve 142 to the open state. The pneumatic controller may apply a positive pressure through the first pneumatic port 112. The applied positive pressure may be sequentially transferred to the first pneumatic channel 111, the metering chamber 102, the metering channel 122, the second valve 142, the mixing channel 123, the first mixing chamber 104a, and the second pneumatic channel 113. In this process, the binding buffer remaining in the mixing channel 123 may be completely discharged to the first mixing chamber 104a.

Then, the valve controller may switch the second valve 142 to the closed state to close all the valves. The pneumatic controller may close both the first pneumatic port 112 and the second pneumatic port 114.

The ultrasonic means may move to the position corresponding to the first mixing chamber 104a and generate ultrasonic waves in the first mixing chamber 104a to mix the sample and the binding buffer. The heating means may move to the position corresponding to the first mixing chamber 104a and apply heat to the first mixing chamber 104a to incubate for a predetermined time.

After a predetermined time has passed and the first washing process is completed, the magnetic means may move to the position corresponding to the first mixing chamber 104a and generates the electromagnetic field in the first mixing chamber 104a to collect the magnetic beads.

### [Drain process]

Thereafter, a drain process of the first mixing chamber 104a may be performed.

The valve controller may switch the third valve 143 to the open state to open the second waste channel 126. The pneumatic controller may apply negative pressure through the first pneumatic port 112 or positive pressure through the second pneumatic port 114. The applied negative pressure may be sequentially transferred to the first pneumatic channel 113, the first waste channel 125, the waste chamber 103, the second waste channel 126, the third valve 143, the mixing channel 123, and the first mixing chamber 104a.

In this process, a waste fluid in the first mixing chamber 104a is dumped into the waste chamber 103 through the mixing channel 123, the third valve 143, and the second waste channel 126. In this case, the magnetic bead remains attached to the magnetic means.

### [First washing process]

Thereafter, a first washing buffer is transferred to the first mixing chamber 104a and a first magnetic bead washing process may proceed.

The valve control unit may close all valves by switching the third valve 143 to a closed state. The pneumatic controller may shut off the first pneumatic port 112 and apply negative pressure through the second pneumatic port 114. The applied negative pressure may be sequentially transferred to the second pneumatic channel 113, the first mixing chamber 104a, and the second buffer channel 132.

The blister pressurizing means may simultaneously pressurize the first washing buffer chamber 163 to supply the washing buffer. In this process, the first washing buffer of the first washing buffer chamber 163 is transferred to the first mixing chamber 104a through the second buffer channel 132.

The magnetic beads attached to the magnetic means fall off when the magnetic means stops generating the electromagnetic field. The ultrasonic means moves to the position corresponding to the first mixing chamber 104a and generates ultrasonic waves in the first mixing chamber 104a to mix the magnetic beads and the first washing buffer. The heating means moves to the position corresponding to the first mixing chamber 104a and heat is applied to the first mixing chamber 104a to incubate for a predetermined time.

After a predetermined time has passed and the first washing process is completed, the magnetic means may move to the position corresponding to the first mixing chamber 104a and generates the electromagnetic field in the first mixing chamber 104a to collect the magnetic beads.

Thereafter, a first drain process of the first mixing chamber 104a may be performed.

The valve controller may switch the third valve 143 to the open state to open the second waste channel 126. The pneumatic controller may apply negative pressure through the first pneumatic port 112 or positive pressure through the second pneumatic port 114. The applied negative pressure may be sequentially transferred to the first pneumatic channel 113, the first waste channel 125, the waste chamber 103, the second waste channel 126, the third valve 143, the mixing channel 123, and first the mixing chamber 104a.

In this process, the waste fluid in the first mixing chamber 104a is dumped into the waste chamber 103 through the mixing channel 123, the third valve 143, and the second waste channel 126. In this case, the magnetic bead remains attached to the magnetic means.

### [Second washing process]

Thereafter, a second washing buffer is transferred to the first mixing chamber 104a and a second magnetic bead washing process may proceed.

The valve control unit may close all valves by switching the third valve 143 to a closed state. The pneumatic controller may shut off the first pneumatic port 112 and apply negative pressure through the second pneumatic port 114. The applied negative pressure may be sequentially transferred to the second pneumatic channel 113, the first mixing chamber 104a, and the second buffer channel 132.

The blister pressurizing module may simultaneously pressurize the second washing buffer chamber 164 to supply the washing buffer. In this process, the second washing buffer of the second washing buffer chamber 164 is transferred to the first mixing chamber 104a through the second buffer channel 132.

The magnetic beads attached to the magnetic means fall off when the magnetic means stops generating the electromagnetic field. The ultrasonic means moves to the position corresponding to the first mixing chamber 104a and generates ultrasonic waves in the first mixing chamber 104a to mix the magnetic beads and the second washing buffer. The heating means moves to the position corresponding to the first mixing chamber 104a and heat is applied to the first mixing chamber 104a to incubate for a predetermined time.

After a predetermined time has passed and the second washing process is completed, the magnetic means may move to the position corresponding to the first mixing chamber 104a and generates the electromagnetic field in the first mixing chamber 104a to collect the magnetic beads.

Thereafter, a second drain process of the first mixing chamber 104a may be performed. The valve controller may switch the third valve 143 to the open state to open the second waste channel 126. The pneumatic controller may apply negative pressure through the first pneumatic port 112 or positive pressure through the second pneumatic port 114. The applied negative pressure may be sequentially transferred to the first pneumatic channel 113, the first waste channel 125, the waste chamber 103, the second waste channel 126, the third valve 143, the mixing channel 123, and the first mixing chamber 104a.

In this process, the waste fluid in the first mixing chamber 104a is dumped into the waste chamber 103 through the mixing channel 123, the third valve 143, and the second waste channel 126. In this case, the magnetic bead remains attached to the magnetic means.

Meanwhile, all or part of the second magnetic bead washing process may be omitted depending on circumstances.

Even after the first mixing chamber 104a is emptied, pneumatic pressure may be continuously applied to dry the mixing channel 123 and the second waste channel 126. This is to prevent contamination by reagents or buffers remaining in the mixing channel 123 and the second waste channel 126 in the subsequent elution process.

### [Elution process]

Thereafter, an elution process may be performed in the first mixing chamber 104a by transferring the elution buffer to the first mixing chamber 104a.

The valve control unit may close all valves by switching the third valve 143 to a closed state. The pneumatic controller may shut off the first pneumatic port 112 and apply negative pressure through the second pneumatic port 214. The applied negative pressure may be sequentially transferred to the second pneumatic channel 113, the first mixing chamber 104a, and the third buffer channel 133.

The blister pressurizing means may simultaneously pressurize the elution buffer chamber 165 to supply the elution buffer. In this process, the elution buffer of the elution buffer chamber 165 is transferred to the first mixing chamber 104a through the third buffer channel 133.

After a predetermined time has passed, the pneumatic controller may apply negative pressure again through the second pneumatic port 114 to vent the evaporated gas in the first mixing chamber 104a.

The magnetic beads attached to the magnetic means fall off when the magnetic means stops generating the electromagnetic field. The ultrasonic means moves to the position corresponding to the first mixing chamber 104a and generates ultrasonic waves in the first mixing chamber 104a to mix the magnetic beads and the elution buffer.

The pneumatic controller may block the first pneumatic port 112 and apply positive pressure through the second pneumatic port 114. Since the valves are all closed, the positive pressure may increase the pressure in the first mixing chamber 104a. The heating means may move to the position corresponding to the first mixing chamber 104a and apply heat to the first mixing chamber 104a to increase the temperature within the chamber.

As such, the incubating process may be performed for a predetermined time while the pressure and temperature of the first mixing chamber 104a are increased.

### [Master mix Process]

Thereafter, a master mix process may be performed in which the eluate from the first mixing chamber 104a to the second mixing chamber 104b is transferred and mixed with the master mix. The master mix may include dry reagent. For example, the master mix may include lyophilized beads.

The master mix may be prepared in various combinations according to the type of reaction occurring in the detection chamber 105. For example, the master mix may be prepared in a combination including one or more of DNA polymerase, DNA glycosylase, dNTPs, reference dye, and buffer.

The valve controller may switch the fourth valve 144 to an open state. The pneumatic controller may apply negative pressure through the first pneumatic port 112 or positive pressure through the second pneumatic port 114.

The first pneumatic channel connected to the first pneumatic port 112 is branched into a first branch channel 111a and a second branch channel 111b. The first branch channel 111a may be connected to the second mixing chamber and the second branch channel 111b may be connected to the first waste channel 125.

The first mixing chamber 104a is connected to the second mixing chamber 104b through the mixing connection channel 127 and the fourth valve 144. In this process, the eluate of the first mixing chamber 104a is transferred to the second mixing chamber 104b through the master mix channel 227.

The valve controller may switch the fourth valve 244 to the closed state. The ultrasonic means may generate ultrasonic waves in the second mixing chamber 104b to mix the eluate and the master mix. The heating means may apply heat to the second mixing chamber 104b and incubate for a predetermined time.

After a predetermined time has passed, the pneumatic controller may apply negative pressure through the first pneumatic port 112 to vent the evaporated gas in the second mixing chamber 104b.

### [Detection process]

Thereafter, a pre-detection process of transferring the solution mixed with the master mix in the second mixing chamber 104b to the detection chamber 105 may be performed.

The valve controller may switch the fifth valve 145 to the open state to open the detection channel 135. The pneumatic controller may apply positive pressure through the first pneumatic port 112 while the second pneumatic port 114 is blocked. The applied positive pressure may be sequentially transferred to the first pneumatic channel 111, the second mixing chamber 104b, and the detection channel 135. In this process, the solution mixed with the master mix in the second mixing chamber 104b may be transferred to the detection chamber 105.

Then, an amplification reaction may occur in the detection chamber 105 and a detection process may proceed.

The valve controller may switch the fifth valve 145 to the closed state. The solution transferred from the second mixing chamber 104b to the detection chamber 105 through the detection channel 135 may dissolve the detection reagent beads in the detection chamber 105.

The detection chamber heating means may apply heat to the detection chamber 105 to perform a PCR reaction for a predetermined time. The PCR reaction may be performed at a predetermined temperature for a predetermined time and a plurality of reactions may proceed at two or more temperatures.

The pressure increased while the detection chamber 105 is heated may be released to the detection buffer chamber 106. For example, the evaporated gas in the detection chamber 105 may move to the detection buffer chamber 106.

Then, if the detection reaction is completed, the detection module detects the target analyte in the detection chamber 105. For example, the detection module may emit excitation light incident on the detection chamber 105 and receive emission light reflected from the target analyte.

Finally, before the cartridge 10 on which the detection process is completed is removed from the detection device, a process of releasing the pressure of the cartridge 10 may be performed. The pneumatic controller may apply negative pressure through the first pneumatic port 112. The valve controller may switch the fifth valve 145 to the open state to open the detection channel 135. In this process, the gas of the detection chamber 105 and the detection channel 135 is vented to the first pneumatic port 112.

### [Cartridge removal]

After all the reactions are finished, the valve controller may switch all the valves to the open state. The pneumatic controller opens the first and second pneumatic ports 112 and 114.

The cartridge 10 may be removed from the detection device.

Hereinafter, configurations of the cartridge will be described in more detail.

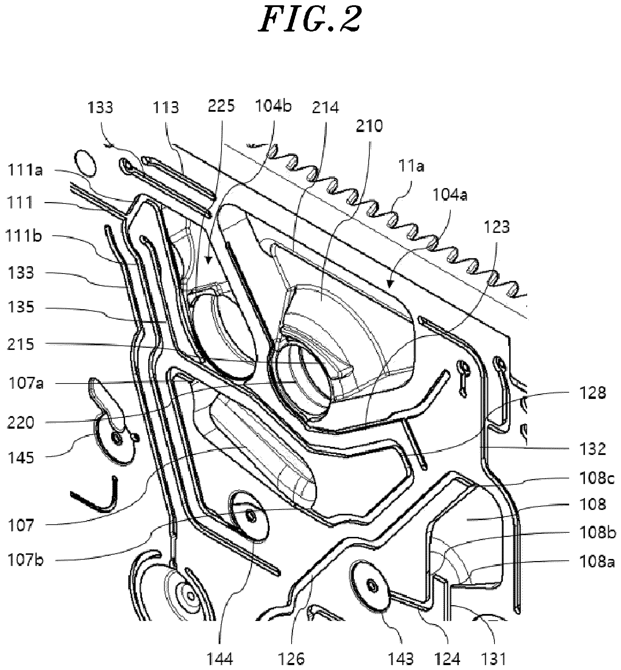

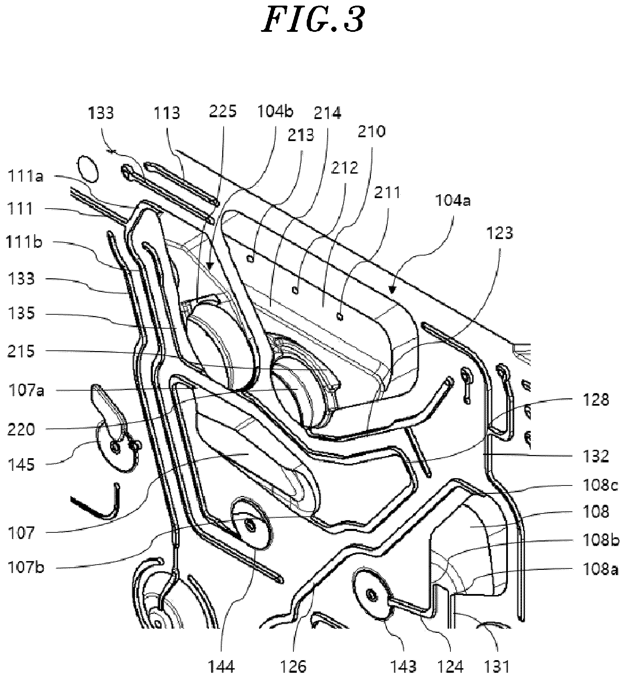

FIG. 2 is a perspective view illustrating a top view of a portion of the cartridge, and FIG. 3 is a perspective view illustrating a bottom view of FIG. 2. Hereinafter, it will be described with reference to FIGS. 2 and 3.

### [First Mixing Chamber]

The first mixing chamber 104a is concavely recessed in the first surface of the base 11 to form a concave surface (or bottom surface) adjacent to the second surface of the base 11. The opening of the first mixing chamber 104a may be sealed with a cover attached to the first surface of the base 11. Hereinafter, the depth represents the average distance in the direction from the first surface of the base to the second surface. The depth of the first mixing chamber 104a represents the average distance from the first surface of the base 11 to the concave surface.

The first mixing chamber 104a may be divided into an upper space 210 and a lower space 220. The upper space 210 and the lower space 220 may be spatially separated while being fluidly connected to each other.

Both sides of the upper space 210 of the first mixing chamber 104a are inclined toward the lower space. The left and right sides of the first mixing chamber 104a may have a shape in which the width of the upper space 210 decreases toward the bottom by being inclined in a direction facing each other.

The lower space 220 of the first mixing chamber 104a may have a cylindrical well or a puddle. For example, the lower space 220 may include a circular concave surface and a cylindrical side surface. Or the lower space 220 may include the puddle of various shapes.

A channel communicating with the upper space 210 may be formed on one upper side of the lower space 220. For example, a side surface of the lower space 220 may form a channel by including a portion having a low height from the concave surface at a portion where the side surface of the upper space 210 is connected.

The upper space 210 may include a hole 211 communicating with the second buffer channel 132 through which the first and second washing buffers are introduced, and a hole 212 communicating with the third buffer channel 133 through which the elution buffer is introduced, and a hole 213 communicating with the second pneumatic channel 113 through which air pressure is transmitted.

A portion of the channels 132, 133, and 113 communicating with the upper space 210 may be formed on the first surface of the base 11. The channels 132, 133, and 113 may pass through the base 11 at a portion adjacent to the first mixing chamber 104a, and then extend adjacent to the rear surface of the upper space 210 along the second surface of the base 11. The channels 132, 133, and 113 may penetrate toward the upper space 210 from the second surface of the base 11 to form holes in the concave surface of the upper space 210. For example, the liquid transport channel 132, 133 connected to the concave surface of the first mixing chamber 104a may comprise a first path provided on the first surface of the base 11, a third path provided on the second surface of the base 11, and a second path penetrating the base 11 to connect the first path and the third path. Additionally, the liquid transport channel 132, 133 connected to the concave surface of the first mixing chamber 104a further comprises a fourth path penetrating the base 11 to connect the third path with the concave surface of the first mixing chamber 104a.

A hole communicating with the mixing channel 123 may be formed at the bottom of the lower space 220. The mixing channel 123 communicating with the lower space 220 may be connected to the lower space 220 along the first surface of the base 11.

The mixing channel 123 may be connected to the lower portion of the first mixing chamber 104a, extend upward, and then diverge toward the shuttle chamber 107 and the ante chamber 108 at the first branch point. At this time, the channel connected to the shuttle chamber 107 at the first branch point is referred to as a mixing connection channel 127, and the channel from the first branch point to the second branch point located in front of the ante chamber 108 is referred to as a mixing channel 123.

The mixing channel 123 branched in the direction of the ante chamber 108 (or in the direction of gravity) at the first branch point may branch into the metering channel 122 and the ante channel 124 at the second branch point. At this time, the channel connected to the metering chamber 102 through the second valve 142 at the second branch point is referred to as the metering channel 122, and the channel connected to the ante chamber 108 through the third valve 143 at the second branch point is referred to as an ante channel 124.

Specifically, the mixing channel 123 is connected upwardly to the mixing connection channel 127 from the first branch point located on the right side of the first mixing chamber 104a and extends downward toward the second branch point. The mixing channel 123 extending downward from the first branch point diverges from the second branch point and is connected to the metering channel 122 to the right and to the ante channel 124 downward.

The first branch point is located above the second branch point and is located closer to the first mixing chamber 104a.

The mixing channel 123 may include a siphon shape. The mixing channel 123 may include a shape that rises upward in the direction of gravity from the bottom of the first mixing chamber 104a and then turns clockwise at the first branch point to descend downward. The first branch point of the mixing channel 123 may be located at the high point of the siphon shape.

The mixing connection channel 127 may include a siphon shape. The mixing connection channel 127 may include a shape that rises upward in the direction of gravity at the first branch point of the mixing channel 123 and then turns clockwise to descend downward. A first branch point of the mixing channel 123 may be located at the front end of the siphon shape of the mixing connection channel 127, and a fourth valve 144 may be located at the rear end of the siphon shape of the mixing connection channel 127.

In the discloser, a case in which the channel includes a siphon shape may include a case in which the channel includes a siphon valve. The siphon valve may not individually stop fluid flow but be used to inhibit fluid flow in the reverse direction without a separate valve structure. The operation of the siphon valve may be determined according to level of capillary force, gravity, and air pressure provided to the channel.

The first mixing chamber 104a may include a structure capable of preventing the mixed solution from splashing into the upper space 210 while a mixing operation occurs in the lower space 220. For example, a barrier wall 215 may be formed in a portion except for a ditch through which fluid flows between the upper space 210 and the lower space 220 of the first mixing chamber 104a.

The barrier wall 215 may be provided apart from one side of the upper space 210, and the ditch through which fluid flows is formed in the space between them. For example, the barrier wall 215 may be provided apart from both sides of the first mixing chamber 104a, and the ditches may be formed on both sides of the barrier wall 215.

Holes 211, 212, and 213 through which fluid flows into the upper space 210 may be formed on the concave surface of the first mixing chamber 104a. When the holes through which the fluid is introduced are formed on the side surface of the first mixing chamber 104a, the hole and the channel connected to the hole may be contaminated by the solution rising along the side surface of the lower space 220 or by the droplets that are broken and bounced in the process of mixing the solution. However, when the holes 211, 212, and 213 through which the fluid is introduced are formed on the concave surface, it can be protected from the solution rising along the side surface and the droplets bouncing off the barrier wall 215.

The upper space 210 may include a step 214 that deepens downward. The step 214 may be provided to be sloped downward so that the fluid introduced from the holes can flow well downward.

The holes 211, 212, and 213 through which the fluid flows into the upper space 210 may be positioned above the step 214 having a relatively shallow depth. Even when the solution overflowing the lower space 220 flows into the upper space 210, the flow of the solution rising along the concave surface to the step 214 is blocked and contamination of the hole in the upper space 210 and the channel connected to the hole can be prevented.

The upper space 210 is connected to the lower space 220 by raising the lower part of the concave surface. At this time, as described above, a barrier wall 215 may be provided at the boundary between the upper space 210 and the lower space 220, and the ditches connected to the lower space 220 may be formed on both sides of the blocking wall 215.

The raised surface connected from the upper space 210 to the lower space 220 may include a guide surface capable of guiding the liquid toward the ditch. For example, the raised surface may include an inclined surface that lowers in a direction adjacent to the ditch.

When the lower space 220 is provided as a cylindrical well, the raised surface may include a fan-shaped hill with a lower depth toward the lower space 220. The liquid flowing downward by gravity on the raised surface may move to both sides of the raised surface and be introduced into the lower space 220 through ditches located on both sides of the barrier wall 215.

The first mixing chamber 104a may further comprising a first bead cap accommodating the magnetic bead, an internal control bead, and a protease K bead. The lower space 220 may be provided to penetrate the second surface of the base 11, and the first bead cap may be inserted and mounted into the lower space 220 on the second surface of the base. The first surface of the first mixing chamber 104a may be sealed with a cover covering the first surface of the base 11.

### [Ante chamber]

The target analyte detection cartridge according to an embodiment of disclosure may include an ante chamber 108 connected to the first mixing chamber 104a and ante channel 124 connecting the first mixing chamber 104a and the ante chamber 108.

The ante chamber 108 may be located between the first mixing chamber 104a and the buffer chamber and may also be located between the first mixing chamber 104a and the waste chamber 103. Hereinafter, the buffer chamber may include a lysis buffer chamber 161 and a binding buffer chamber 162.

One side of the ante chamber 108 may be connected to the first mixing chamber 104a sequentially passing through the ante channel 124 and the mixing channel 123. The third valve 143 may be provided for opening and closing the ante channel 124. However, it is possible to mix and use the ante channel 124 and the mixing channel 123 if necessary, and it is also possible to express that the third valve 143 opens and closes the mixing channel 123.

The ante chamber 108 may be connected to the first buffer channel 131, the ante channel 124, and the second waste channel 126. On one side of the ante chamber 108, the first port 108a connected to the first buffer channel 131 and the second port 108b connected to the ante channel 124 may be provided and disposed adjacent to each other. On other side of the ante chamber 108, the third port 108c connected to the second waste channel 126 may be provided.

The first port 108a and the second port 108b provided on one side of the ante chamber 108 may be disposed on opposite sides of the third port 108c provided on the other side. For example, the first and the second port 108a, 108b may be located relatively lower in the direction of gravity than the third port 108c.

The ante chamber 108 may be concavely recessed in the first surface of the base 11 to form a concave surface (or bottom surface) adjacent the second surface of the base 11. An opening of the ante chamber 108 may be sealed with a cover attached to the first surface of the base 11. The depth of the ante chamber 108 represents the average distance from the cover attached to the first surface of the base 11 to the concave surface.

The ante chamber 108 may include portions with different depths. The ante chamber 108 may be provided so that the depth of one side where the first port 108a and the second port 108b are provided is shallower than the depth of the other side where the third port 108c is provided. The ante chamber 108 may include a slope that increases in depth from one side to the other side.

The ante chamber 108 may include portions with different widths. The ante chamber 108 may have a shape whose width becomes narrower as it approaches the channel. The ante chamber 108 may have a shape that gradually widens in width from one side where the first port 108a and the second port 108b are provided toward the other side where the third port 108c is provided, and then narrows again. That is, the ante chamber 108 may include a portion whose cross-sectional area increases from one side to the other and then decreases again.

The lysis buffer and binding buffer may be introduced into the ante chamber 108 from the buffer chamber through the first buffer channel 131 and discharged from the ante chamber 108 through the ante channel 124. The lysis buffer and binding buffer may flow into the first mixing chamber 104a through the mixing channel 123 connected to the ante channel 124.

The waste fluid drained from the first mixing chamber 104a may sequentially pass through the mixing channel 123 and the ante channel 124 and flow into the ante chamber 108. Then, the waste fluid is discharged from the ante chamber 108 through the second waste channel 126 and discarded into the waste chamber 103.

The third valve 143 may open and close the ante channel 124. A convergence point where the metering channel 122 joins the mixing channel 123 may be provided on one side of the third valve 143.

The ante chamber 108 may capture buffer bubbles discharged from the buffer chamber (e.g., blister chamber). Accordingly, the buffer in the ante chamber 108 may be delivered to the first mixing chamber 104a with bubbles removed.

The ante chamber 108 may prevent the mixing channel 123 from being contaminated by lysis buffer or binding buffer remaining in the mixing channel 123. If lysis buffer or binding buffer remains in the mixing channel 123, the eluate that has completed the elution process may be contaminated as it flows into the second mixing chamber 104b through the mixing connection channel 127 and the shuttle channel 128.

The ante chamber 108 may be used as a pressure buffer space. Specifically, when negative pressure is generated in the first mixing chamber 104a, it is possible to prevent the residual buffer from flowing back from the first buffer channel 131 to the mixing channel 123. And it is possible to prevent the residual buffer from flowing back from the ante chamber 108 to the mixing channel 123 by closing the third valve 143 provided in the ante channel 124.

### [Shuttle chamber]

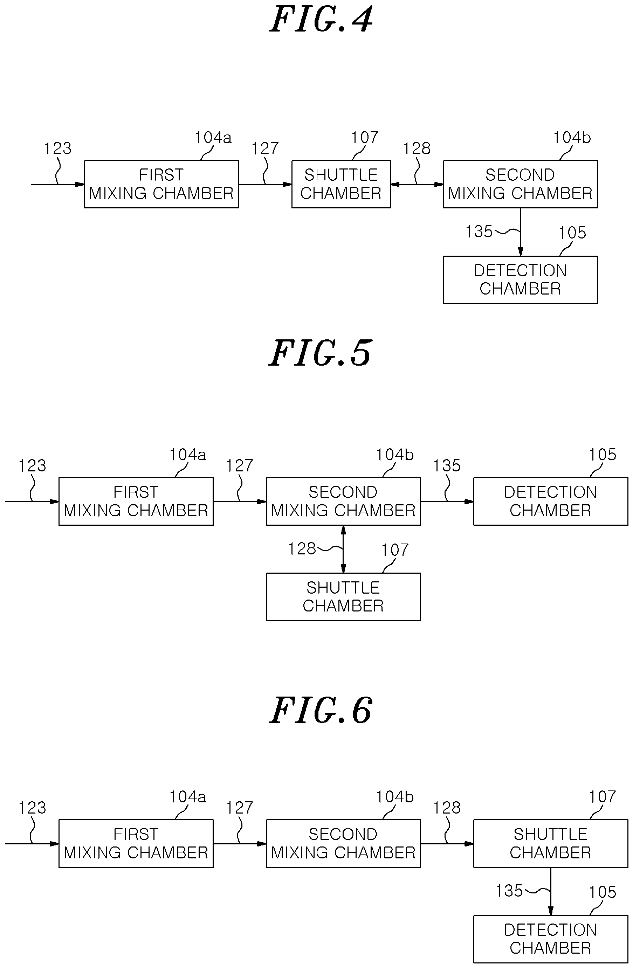

Thereafter, the shuttle chamber 107 is described with reference to FIGS. 4 to 6.

The target analyte detection cartridge according to an embodiment of the disclosure includes a shuttle chamber 107 connected to the second mixing chamber 104b and a shuttle channel 128 connecting to shuttle chamber 107 and the second mixing chamber 104b. The shuttle chamber 107 may be disposed between the first mixing chamber 104a and the second mixing chamber 104b in terms of fluid flow.

The shuttle chamber 107 may be used to dissolve the master mix inside the second mixing chamber 104b in the eluate supplied through the shuttle channel 128. For example, the master mix in the solution inside the second mixing chamber 104b may be dissolved while moving to or returning from the shuttle chamber 107.

Ultrasonic wave may be applied as in the first mixing chamber 104a to dissolve the master mix in the second mixing chamber 104b in the eluate. However, the use of ultrasonic wave under conditions exceeding a certain level may cause the problem of impairing the stability of enzymes within the master mix. For example, the problem may be that the stability of enzymes including DNA polymerase within the master mix is impaired.

Therefore, when using ultrasonic wave to dissolve the master mix in the eluate, it is required to use it within a certain range of conditions, and if these conditions are not met, dissolution of the master mix may become insufficient. Alternatively, it may be necessary to dissolve the master mix in the eluate without using ultrasound to ensure enzyme stability within the master mix.

The shuttle chamber 107 may be connected to the second mixing chamber 104b through a shuttle channel 128. And the solution in the second mixing chamber 104b (e.g., a solution containing an undissolved dried master mix and a dissolved master mix) may move to the shuttle chamber 107 through the shuttle channel 128. Then, the solution in the shuttle chamber 107 may return to the second mixing chamber 104b through the shuttle channel 128 after a certain period of time or immediately. Furthermore, the solution in the second mixing chamber 104b may move back and forth through the shuttle chamber 107 two or more times.

FIG. 4 is a structural diagram illustrating a shuttle chamber arrangement according to an embodiment of the disclosure.

Referring to FIG. 4, one side of the shuttle chamber 107 may be connected to the first mixing chamber 104a through the mixing connection channel 127, and the other side may be connected to the second mixing chamber 104b through the shuttle channel 128. At this time, the mixing connection channel 127 may be referred to as including the mixing channel 123.

The eluate in the first mixing chamber 104a may move to the shuttle chamber 107 through the mixing connection channel 127 and then may move to the second mixing chamber 104b through the shuttle channel 128. The solution may move from the second mixing chamber 104b to the shuttle chamber 107 through the shuttle channel 128 and then back to the second mixing chamber 104b to dissolve the master mix provided in the second mixing chamber 104b. This reciprocating flow of solution may be performed out multiple times.

Fluid flow between the second mixing chamber 104b and the shuttle chamber 107 may be generated by pneumatic pressure. For example, positive pressure may be provided to the second mixing chamber 104b or negative pressure may be provided to the first mixing chamber 104a so that the fluid in the second mixing chamber 104b may move into the shuttle chamber 107. And negative pressure is provided to the second mixing chamber 104b or positive pressure is provided to the first mixing chamber 104a so that the fluid in the shuttle chamber 107 may return to the second mixing chamber 104b.

In this case, a separate pneumatic channel may not be provided in the shuttle chamber 107. The shuttle chamber 107 may indirectly transmit the pneumatic pressure provided to the first mixing chamber 104a, and the pneumatic pressure provided to the second mixing chamber 104b may be indirectly transmitted. Herein, the meaning that pneumatic pressure is transmitted indirectly may include the meaning that pneumatic pressure is transmitted through an adjacent chamber.

FIG. 5 is a structural diagram illustrating the shuttle chamber arrangement according to another embodiment of the disclosure.

Referring to FIG. 5, the shuttle chamber 107 may be connected to the second mixing chamber 104b through a shuttle channel 128 provided separately from the mixing connection channel 127 connecting the first mixing chamber 104a and the second mixing chamber 104b. For example, one side of the second mixing chamber 104b may be connected to the first mixing chamber 104a through the mixing connection channel 127, and the other side may be connected to the shuttle chamber 107 through the shuttle channel 128. At this time, the mixing connection channel 127 may be used to mean included in the mixing channel 123.

The eluate in the first mixing chamber 104a may move to the second mixing chamber 104b through the mixing connection channel 127. The solution may move from the second mixing chamber 104b to the shuttle chamber 107 through the shuttle channel 128 and then back to the second mixing chamber 104b to dissolve the master mix provided in the second mixing chamber (104b. This reciprocating flow of solution may be performed out multiple times.

Fluid flow between the second mixing chamber 104b and the shuttle chamber 107 may be generated by pneumatic pressure. For example, positive pressure may be provided to the second mixing chamber 104b or negative pressure may be provided to the shuttle chamber 107 so that the fluid in the second mixing chamber 104b may move into the shuttle chamber 107. And negative pressure is provided to the second mixing chamber 104b or positive pressure is provided to the shuttle chamber 107 so that the fluid in the shuttle chamber 107 may return to the second mixing chamber 104b.

In this case, a pneumatic channel may be provided in the shuttle chamber 107. The fluid may be delivered to the second mixing chamber 104b by providing positive pressure directly to the shuttle chamber 107, and the fluid may be drawn in the second mixing chamber 104b by providing negative pressure directly to the shuttle chamber 107.

FIG. 6 is a structural diagram illustrating the shuttle chamber arrangement according to another embodiment of the disclosure.

Referring to FIG. 6, the shuttle chamber 107 may be located between the second mixing chamber 104b and detection chamber 105. One side of the shuttle chamber 107 may be connected to the second mixing chamber 104b through the shuttle channel 128, and the other side may be connected to the detection chamber 105 through the detection channel 135. At this time, the mixing connection channel 127 may be used to mean included in the mixing channel 123.

The eluate in the first mixing chamber 104a may move to the second mixing chamber 104b through the mixing connection channel 127. The solution may move from the second mixing chamber 104b to the shuttle chamber 107 through the shuttle channel 128 and then back to the second mixing chamber 104b to dissolve the master mix provided in the second mixing chamber 104b. This reciprocating flow of solution may be performed out multiple times.

Fluid flow between the second mixing chamber 104b and the shuttle chamber 107 may be generated by pneumatic pressure. For example, positive pressure may be provided to the second mixing chamber 104b or negative pressure may be provided to the shuttle chamber 107 so that the fluid in the second mixing chamber 104b may move into the shuttle chamber 107. And negative pressure is provided to the second mixing chamber 104b or positive pressure is provided to the shuttle chamber 107 so that the fluid in the shuttle chamber 107 may return to the second mixing chamber 104b.

In this case, a pneumatic channel may be provided in the shuttle chamber 107. The fluid may be delivered to the second mixing chamber 104b by providing positive pressure directly to the shuttle chamber 107, and the fluid may be drawn in the second mixing chamber 104b by providing negative pressure directly to the shuttle chamber 107.

The dissolved solution may be moved directly from the shuttle chamber 107 to the detection chamber 105.

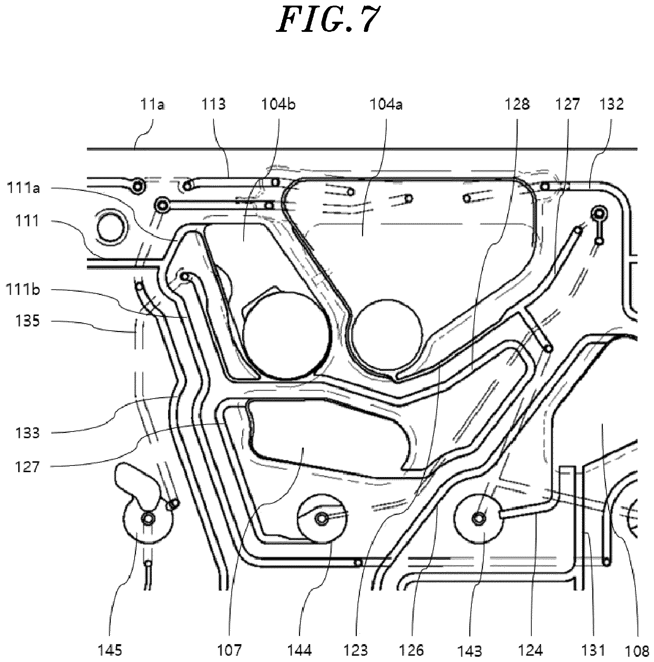

FIG. 7 is an enlarged view for explaining the shuttle chamber according to an embodiment of the disclosure.

Referring to FIG. 7, one side of the shuttle chamber 107 may be connected to the first mixing chamber 104a through the mixing connection channel 127 and mixing channel 123, and the other side may be connected to the second mixing chamber 104b through the shuttle channel 128. The fourth valve 144 may be provided opening and closing the mixing connection channel 127. At this time, the mixing connection channel 127 may be referred to as a meaning distinguished from the mixing channel 123.

The shuttle chamber 107 may be disposed below the first mixing chamber 104a and the second mixing chamber 104b. Specifically, the second mixing chamber 104b may be located to the left of the first mixing chamber 104a, and the shuttle chamber 107 may be located below the first mixing chamber 104a and the second mixing chamber 104b. The mixing connection channel 127 passes under the shuttle chamber 107 from the right side of the first mixing chamber 104a and is bent clockwise to be connected to the upper left side of the shuttle chamber 107. The shuttle channel 128 may extend upward from the lower right side of the shuttle chamber 107 and be bent counterclockwise to be connected to the lower right side of the second mixing chamber 104b. The shuttle channel 128 may be connected to the lowermost part of the shuttle chamber 107. The mixing connection channel 127 may be connected to the uppermost part of the shuttle chamber 107.

The shuttle channel 128 may include a siphon shape. The shuttle channel 128 may include a shape that rises upward from the lower right side of the shuttle chamber 107 and then turns counterclockwise to descend downward. The shuttle chamber 107 may be located at front end of the siphon shape of the shuttle channel 128, and the second mixing chamber 104b may be located at the rear end of the siphon shape.

The shuttle chamber 107 may be located between the second mixing chamber 104b and the fourth valve 144 in the vertical direction. The shuttle chamber 107 may be located between the second waste channel 126 and the third buffer channel 133 in the horizontal direction. Or the shuttle chamber 107 may be located between the second waste channel 126 and the detection channel 135 in the horizontal direction.

The shuttle chamber 107 may be provided in a shape where the width in the horizontal direction is greater than the height in the vertical direction. The mixing connection channel 127 may be connected to the left side of the shuttle chamber 107, and the shuttle channel 128 may be connected to the right side of the shuttle chamber 107. The mixing connection channel 127 may be connected above the shuttle chamber 107, and the shuttle channel 128 may be connected below the shuttle chamber 107. The first port 107a (see FIG. 3) of the shuttle chamber 107 connected to the mixing connection channel 127 may be positioned higher in the vertical direction than the second port 107b (see FIG. 3) of the shuttle chamber 107 connected to the shuttle channel 128.

The solution flowing through the fourth valve 144 and the first port 107a of the shuttle chamber 107 fills the shuttle chamber 107 from the bottom of the shuttle chamber 107. Therefore, backflow toward the fourth valve 144 may be prevented before the shuttle chamber 107 is filled.

The solution filled in the shuttle chamber 107 may be discharged into the shuttle channel 128 through the second connector 107b located below the shuttle chamber 107. At this time, bubbles contained in the solution may be discharged into the space above the shuttle chamber 107, and the solution flowing into the second mixing chamber 104b through the shuttle channel 128 may have its bubbles removed.

The shuttle chamber 107 may be concavely recessed in the first surface of the base 11 to form a concave surface (or bottom surface) adjacent the second surface of the base 11. The opening of the shuttle chamber 107 may be sealed with a cover attached to the first surface of the base 11. The depth of the shuttle chamber 107 represents the average distance from the cover attached to one surface of the base 11 to the concave surface.

The shuttle chamber 107 may include portions with different depths. The depth of one side where the first port 107a is provided may be deeper than the depth of the other side where the second port 107b is provided. The shuttle chamber 107 may include a slope whose depth becomes shallower as it goes from one side to the other. For example, the shuttle chamber 107 may include a portion where the depth of the concave surface increases as it moves away from the second port 107b.

### [Second mixing chamber]

The second mixing chamber 104b is concavely recessed in the first surface of the base 11 to form a concave surface (or bottom surface) adjacent to the second surface of the base 11. The opening of the second mixing chamber 104b may be sealed with a cover attached to the first surface of the base 11. The depth of the second mixing chamber 104b represents the average distance from the first surface of the base 11 to the concave surface.

The second mixing chamber 104b may be located to the left of the first mixing chamber 104a. A portion of the second mixing chamber 104b where bead dissolution occurs may be located adjacent to the portion of the first mixing chamber 104a where bead dissolution occurs. When the cartridge is mounted on the detection device, the ultrasonic means and the heating means may be disposed on the rear of the cartridge. At this time, the ultrasonic means and the heating means may be provided to be movable in the horizontal direction of the cartridge. Accordingly, the ultrasonic means or the heating means may act on the first mixing chamber 104a and then move a predetermined distance to act on the second mixing chamber 104b.

The second mixing chamber 104b may be divided into an upper space and a lower space. The upper space and the lower space may be spatially separated while being fluidly connected to each other. The upper space is connected to the first pneumatic channel 111 to provide pneumatic pressure, and the lower space accommodates the master mix and dissolution of the master mix occurs.

The lower space of the second mixing chamber 104b may have a cylindrical well shape. For example, the lower space may include a circular concave surface and a cylindrical side surface.

A channel communicating with the upper space may be formed on one upper side of the lower space. For example, a side surface of the lower space may form a channel by including a portion having a low height from the concave surface at a portion where the side surface of the upper space is connected.

The upper space is in communication with the first pneumatic channel 111 through a port so that the pneumatic pressure may be transmitted. The port to which the first pneumatic channel is connected is located furthest from the lower space to prevent contamination with the solution. Specifically, the second mixing chamber 104b may be connected to the first pneumatic channel 111 through a port located at the upper left corner. Additionally, the upper space of the second mixing chamber 104b may include a shape that protrudes convexly toward the inside of the left side, thereby preventing the solution in the lower space from rising along the left side.

The second mixing chamber 104b may further comprising a second bead cap accommodating the dry reagent. The lower space may be provided to penetrate the second surface of the base 11, and the second bead cap may be installed by inserting into the lower space from the second surface of the base. The first surface of the second mixing chamber 104b may be sealed with a cover covering the first surface of the base 11.

The shuttle channel 128 and the detection channel 135 may be respectively connected to the bottom of the lower space. For example, at the bottom of the second mixing chamber 104b, the shuttle channel 128 may be connected to the right and the detection channel 135 may be connected to the left. The shuttle channel 128 and the detection channel 135 communicating with the lower space may be connected to the lower space along the first surface of the base 11.

The detection channel 135 may include a siphon shape. The detection channel 135 may include a shape that rises upward from the lower part of the second mixing chamber 104b and then turns counterclockwise to descend downward. The second mixing chamber 104b may be located at front end of the siphon shape of the detection channel 128, and the fifth valve 145 may be located at the rear end of the siphon shape.

The second mixing chamber 104b may include a structure capable of preventing the mixed solution from splashing into the upper space while a mixing operation occurs in the lower space. For example, a barrier wall 225 may be formed in a portion except for a ditch through which fluid flows between the upper space and the lower space of the second mixing chamber 104b. The barrier wall 225 may be provided apart from one side of the upper space, and the ditch through which fluid flows is formed in the space between them. For example, the barrier wall 225 may be provided apart from both sides of the second mixing chamber 104b, and the ditches may be formed on both sides of the barrier wall 225.

The first pneumatic channel 111 may be connected to the upper space along the first surface of the base 11. Therefore, even if the solution overflowing the lower space flows into the upper space, the depth becomes deeper as it goes upward, thereby preventing contamination of the connector located on the first side of the upper space.

### [Sample chamber]

A sample chamber according to an embodiment of the disclosure may be provided to inject a sample quickly and safely into a thin plate-shaped cartridge.

In the conventional method, a metering process had to be performed manually outside the cartridge before injecting the sample into the cartridge. The operator manually measured a fixed amount of sample using a pipette and injected the entire amount of the measured sample. However, this method has problems in that it takes a lot of time and effort to quantify using a pipette, bubbles may be generated in the process of injecting the entire measured sample, and extra samples remain in the sample chamber, etc.

According to an embodiment of the disclosure, even if an arbitrary amount of sample exceeding the minimum volume is injected, quantification is possible through the metering chamber in the cartridge, so there is no need for an operator to manually perform quantification. And because extra sample could be left in the pipette, the problem of bubbles occurring due to air being injected into the sample chamber during the injection process may be solved. Additionally, a marker or an indicator may be placed in the sample chamber to visually confirm whether the minimum volume has been injected.

It is advantageous to have a larger opening diameter of the injection hole, as it allows for a quick and safe sample injection. When an operator injects a sample into a cartridge using a pipette, if the opening diameter of the injection hole is small, leakage may occur, and difficulty and time of work may increase. And the sample could be flowed with the help of gravity to quickly inject the sample. Additionally, bubbles should not be generated during the sample injection process, and it would be good if the amount of sample injected could be visually confirmed.

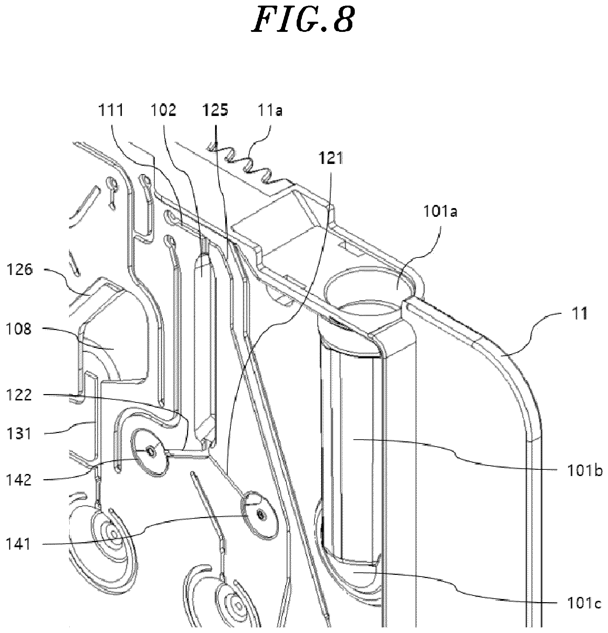

Hereinafter, the sample chamber 101 will be described in detail with reference to FIGS. 8 to 11.

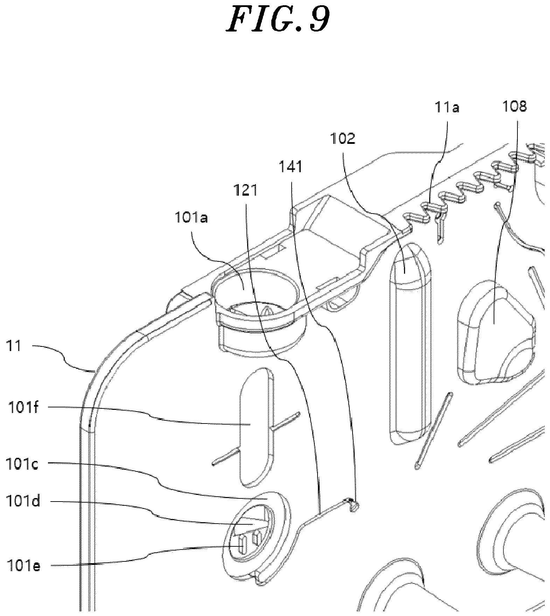

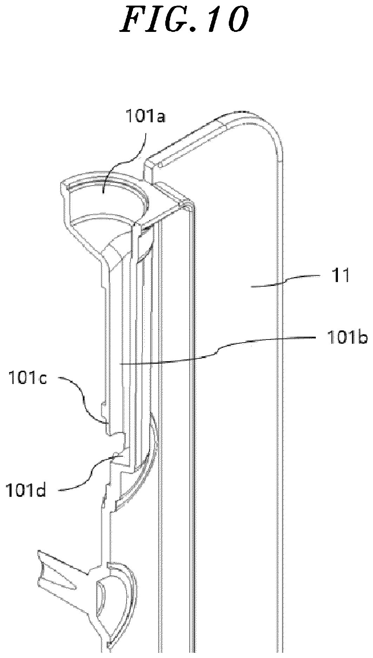

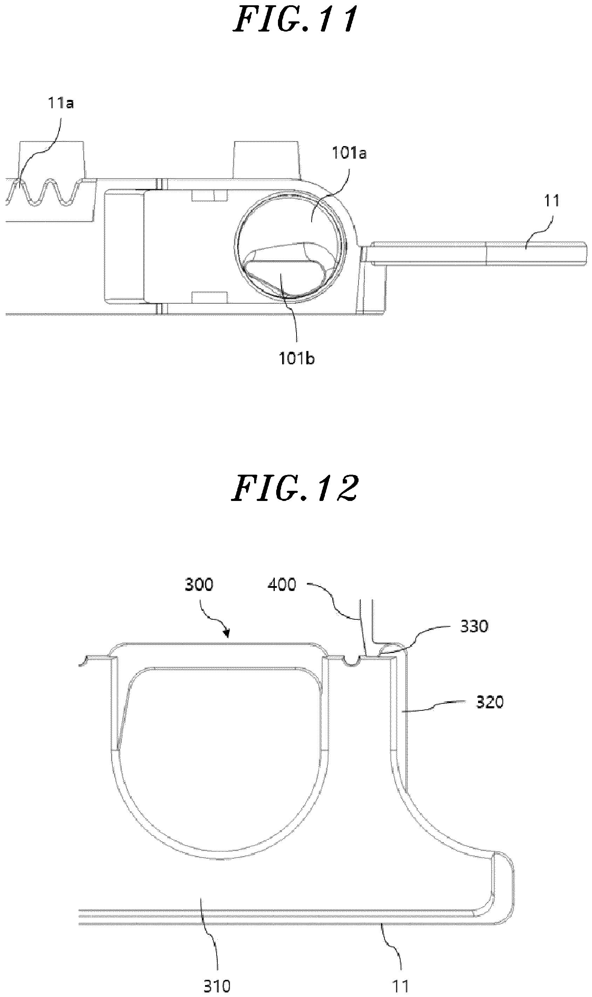

FIG. 8 and 9 are a perspective view illustrating a front view and a rear view of a sample chamber according to an embodiment of the disclosure. FIG. 10 is a vertical cross-sectional view and FIG. 11 is an upper side view for explaining the sample chamber.

According to an embodiment of the disclosure, the sample chamber 101 may include an injection hole 101a through which a sample is injected and storage space 101b and 101c into which the injected sample is stored. The storage space may include a sample delivery passage 101b connected to the injection hole 101a and delivering the sample in the direction of gravity, and a sample filter or sample filter mounting portion 101c provided between the outlet of the sample delivery passage 101b and the sample channel 121. Although the sample filter is omitted in the drawing, a circular sample filter may be mounted on the sample filter mounting portion 101c.

Referring to the drawings, the sample chamber 101 may be positioned at the right side of the upper edge of the base 11 so that the sample could be injected. Hereinafter, the edge refers to a side surface of the plate-shaped base 11 excluding the first surface and the second surface having a large area.

Both the rack 11a and the injection hole 101a may be provided on the upper side surface of the base 11 so as not to overlap. For example, the injection hole 101a may be located on the right side of the rack 11a used for entry and exit of the cartridge 10. And the surface on which the injection hole 101a is formed may be located below the surface on which the rack 11a is formed. Therefore, it is possible to prevent the injection hole 101a from interfering with the rack 11a while the cartridge 10 is inserted and mounted to the left.

And the injection hole 101a may be opened and closed by a stopper (not shown). The stopper is formed integrally with the base 11 to prevent loss. The illustration of the stopper is omitted in the drawing.

The upper side of the base 11 where the injection hole 101a is provided may be expanded in the thickness direction. Therefore, the diameter of the injection hole 101a may be provided larger than the thickness of the base 11. The sample delivery passage 101b may also be expanded from the first surface of the base 11 to be larger than the thickness diameter of the base 11.

The cartridge 10 according to one embodiment may be inserted by standing with the first surface of the base 11 facing the front. When the cartridge 10 is inserted, the injection hole 101a is positioned above, and the sample delivery passage 101b extends downward. Accordingly, the sample injected into the injection hole 101a could pass through the sample delivery passage 101b by gravity.

The cross-sectional area of the injection hole 101a may be larger than the cross-sectional area of the sample delivery passage 101b, so that the sample could be quickly and stably injected. The upper portion of the injection hole 101a may have a cylindrical shape with a constant radius, and the lower portion may include a guide surface inclined inward toward the sample delivery passage 101b.

In the sample chamber 101, the injection hole 101a may have a circular cross-section and the sample delivery passage 101b may have an asymmetrically curved cross-section. Furthermore, the largest inner diameter of the sample delivery passage 101b may be smaller than or equal to the inner diameter of the injection hole 101a.

The sample delivery passage 101b may have a shape that prevents the sample from being stopped due to adhesion or bubble formation while the sample is being transferred. Specifically, the sample delivery passage 101b may include a portion whose horizontal cross-section is asymmetrical.

The sample delivery passage 101b may include a section in which the meniscus of the flowing sample is asymmetrically formed. For example, the horizontal cross-section of the sample delivery passage 101b may have an asymmetric curved shape. The radius of curvature of the curved surface on one side of the cross-section of the sample delivery passage 101b may be different from the radius of curvature of the curved surface on the other opposite side.

Referring to FIG. 11, the radius of curvature of the right curved surface of the cross-section of the sample delivery passage 101b may be larger than the radius of curvature of the left curved surface. The height of the meniscus on the right side of the sample delivery passage 101b, which has a relatively large radius of curvature, may be formed to be lower than the height of the meniscus on the left side, which has a relatively small radius of curvature. This is due to the reason that the surface tension of the sample is greater on the left side of the sample delivery passage 101b, which has a relatively small radius of curvature, than on the right side, which has a relatively large radius of curvature.

Preferably, a ratio of curvature radius on both sides of the crosssection of the sample delivery passage 101b may be set in the range of 1:2 to 1:2.5. If the ratio of the curvature radius on both sides is less than 1:2, sample transfer may be stopped, or bubbles may be formed during transfer since the difference between the meniscus on both sides is small. In addition, if the ratio of the curvature radius on both sides is greater than 1:2.5, there is a risk of bubbles being formed since the difference in transfer speed between both sides is too large.

In addition, in the cross section of the sample delivery passage 101b, the inner diameter in the horizontal direction may be more than twice as large as the inner diameter in the width direction perpendicular thereto. Preferably, the ratio of the inner diameter in the width direction and the inner diameter in the horizontal direction may be set in the range of 1:2 to 1:2.5. If the ratio of the inner diameter in the width direction and the inner diameter in the horizontal direction is less than 1:2, the difference between the meniscus in the width direction and the horizontal direction is small, so sample transfer may be stopped or bubbles may be formed during transfer. Conversely, if the ratio of the inner diameters in the horizontal direction is greater than 1:2.5, the difference in transfer speed in the width direction and the horizontal direction increases, so there is a risk of bubbles formation.

The sample delivery passage 101b may have the same cross-sectional shape in the longitudinal direction. Furthermore, the sample delivery passage 101b may have a cross-sectional shape that has the same shape and area in the longitudinal direction. Alternatively, unlike the drawings, the sample delivery passage 101b may include a shape that becomes narrower with the smaller area as it goes downward in the longitudinal direction but maintains the same shape.

And referring to FIGS. 1 and 8, the outlet of the sample delivery passage 101b may include an inclined surface 101d in the longitudinal direction. For example, the sample delivery passage 101b may extend downward on a side with a relatively smaller radius of curvature than on a side with a relatively larger radius of curvature. This is because the surface tension of the sample is greater on the side with a smaller radius of curvature, and the fluid velocity is relatively faster there. And the sample could be flown to the sample filter 101c without any remaining amount along the inclined surface 101d provided in the longitudinal direction at the outlet of the sample delivery passage 101b.

The sample delivery passage 101b may include a sample height indicator provided on a transparent or a translucent portion. For example, the second surface (rear surface) of the base 11 may include a transparent or a translucent portion of the sample delivery passage 101b, so that the height of the injected sample may be confirmed after sample injection is completed. For example, the sample height indicator may include a horizontal scale.

Referring to FIG. 9, the sample delivery passage 101b may form an opening in the second surface of the base 11. An inclined surface 101d of the outlet of the sample delivery passage 101b may be located inside of the opening of the sample delivery passage 101b. A sample filter mounting portion 101c on which a sample filter (not shown) is mounted may be provided inside the opening of the sample delivery passage 101b. For example, the sample filter may be provided in a disk shape, and the sample filter mounting portion 101c may be provided as a ring-shaped sheet seat to which the circumferential edge of the sample filter is attached.

The sample chamber 101 may further include a sample filter spacer 101e to ensure fluidity of the sample from the front end to the back end of the sample filter. The sample filter spacer 101e may prevent the sample filter from blocking the outlet of the sample delivery passage 101b and may secure the fluidity of the sample by securing the gap between the sample filter and the second surface of the base 11. A plurality of sample filter spacers 101e may be provided to effectively support the sample filter spaced apart from each other. For example, the sample filter spacer 101e may be a support protrusion located inside the sample filter mounting portion 101c and below the inclined surface 101d of the outlet of the sample delivery passage 101b.

Then, the filtered sample passing through the sample filter in the sample delivery passage 101b is delivered to the sample channel 121 connected to the bottom of sample chamber 101.

A cover may be attached to the second surface of the base 11 to seal the opening. Specifically, a cover may be attached to the second surface of the base 11 to seal the opening for mounting the sample filter and the sample channel 121.

### [Waste chamber]

Hereinafter, the waste chamber 103 (see FIG. 1) will be described in detail with reference to FIGS. 12 to 14.

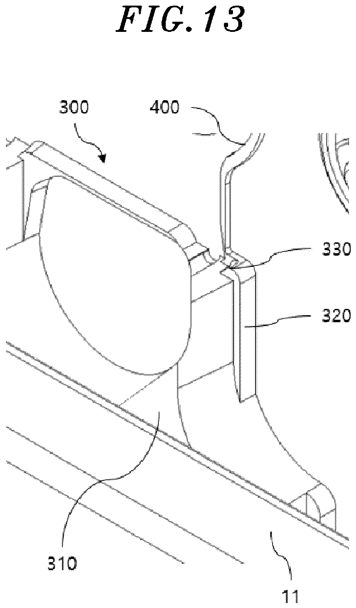

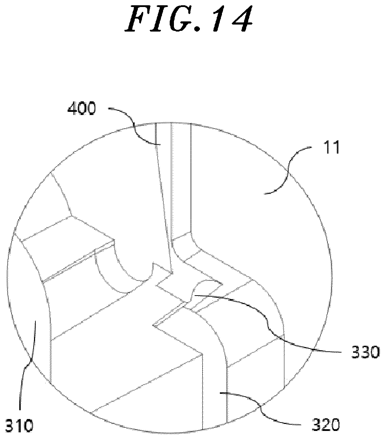

FIG. 12 is a plan view for explaining a waste chamber according to an embodiment of the disclosure. And FIG. 13 is a perspective view illustrating a bottom view of the waste chamber, and FIG. 14 is a partially enlarged view of FIG. 13.

The waste chamber 103 according to an embodiment of the disclosure may be connected to the first waste channel 125 and the second waste channel 126. The sample that overflows the metering chamber 102 is transferred into the waste chamber 103 through the first waste channel 125, and the waste fluid that drains from the first mixing chamber 104a is transferred into the waste chamber 103 through the second waste channel 126.

Hereinafter, the waste chamber 103 will be referred to as a chamber 300, and the first waste channel 125 or the second waste channel 126 connected to the waste chamber 103 will be referred to as an inflow channel 400. And the description of the chamber 300 and the inflow channel 400 described below may be applied to the waste chamber 103, the first waste channel 125, and the second waste channel 126.

The base 11 (see FIG. 1) includes a first surface (front surface), a second surface (back surface), and side surfaces (lateral surfaces) connecting the first surface and the second surface. The side surfaces include an upper surface and a lower surface.

The chamber 300 is formed to be concavely recessed on the first surface of the base 11 and is communicated with the inflow channel 400. The inflow channel 400 is formed to be concavely recessed in the first surface of the base 11. The depth of the chamber 300 is provided to be deeper than the depth of the inflow channel 400.

The chamber 300 may include an accommodation room 310 for receiving the fluid, an inlet port to which the inflow channel 400 is connected, a guide groove that guides the fluid flowing in from the inlet port downward in the direction of gravity of the accommodation room 310, and a barrier structure 330 that prevents the fluid from flowing back from the guide groove 320 toward the inlet port.

The accommodation room 310 may include a ceiling surface and a floor surface (or concave surface) facing each other, an upper wall and a lower wall, and side walls connecting the upper wall and the lower wall.

Here, the ceiling surface and the floor surface of the accommodation room 310 do not refer to the ceiling and floor in the direction of gravity but the ceiling and floor in terms of the shape of the chamber. And the upper wall and the lower wall do not refer to up and down in terms of the shape of the chamber but upward and downward in the direction of gravity.

The ceiling surface of the accommodation room 310 may be a cover that covers the opening formed in the first surface of the base 11. In the drawing, the accommodation room 310 does not penetrate the second surface of the base 11, but the floor surface is shown to be located close to the second surface. Alternatively, unlike the drawing, the accommodation room 310 may be provided to penetrate the second surface of the base 11. In this case, the floor surface of the accommodation room 310 may be a cover that covers the second surface of the base 11.

The cover that seals the ceiling and/or floor of the accommodation room 310 may be provided of a polymer membrane containing a polycarbonate sheeted film or a metal membrane containing an aluminum foil. The cover may be provided of various materials and be formed of a plate shape rather than a membrane. And the cover may be attached to the base 11 by heat compression, adhesive, or mechanical bonding.

When the inlet port is located away from the side wall of the accommodation room 310, the guide groove 320 may include a first portion connected from the inlet port provided on the upper wall of the accommodation room 310 to the adjacent side wall and a second portion extending downward in the direction of gravity along the side wall. Alternatively, when the inlet port is located at a corner where the upper wall and the side wall of the accommodation room 310 meet or is located on the side wall, the first portion may be omitted and the guide groove 320 may extend downward in the direction of gravity along the side wall.

The guide groove 320 may include a floor surface facing the cover and side surfaces provided as part of the upper wall, lower wall, or side wall of the accommodation room 310. The ceiling surface of the guide groove 320 may be part of the cover. The guide groove 320 is open toward the inside of the accommodation room 310. The liquid flowing along the guide groove 320 does not flow toward the inside of the accommodation room 310 due to the adhesive force acting on the three sides of the guide groove 320.

Here, the ceiling surface and the floor surfaces of the guide groove 320 do not refer to the ceiling and floor in the direction of gravity but the ceiling and the floor in terms of the shape of the chamber. And the upper wall and lower wall do not refer to up and down in terms of the shape of the chamber but upward and downward in the direction of gravity.

The depth of the guide groove 320 may include a portion which is deeper than the depth of the inlet port and shallower than the depth of the accommodation room 310. The depth of the guide groove 320 represents the average distance between the ceiling surface and the floor surface.

The cross-sectional area of the guide groove 320 may be set in a range where capillary action of the fluid flowing in through the inflow channel 400 could occur. Accordingly, the liquid flowing into the accommodation room 310 through the inflow channel 400 does not fall below the direction of gravity of the inlet port but could move toward the lower wall of the accommodation room 310 along the guide groove 320. However, the case where some of the liquid flowing into the accommodation room 310 through the inflow channel 400 falls below the direction of gravity of the inlet port is not excluded.

The barrier structure 330 may be provided to reduce fluid resistance from the inlet port toward the guide groove 320. The barrier structure 330 may include a portion where the cross-sectional area of the fluid path increases in the direction from the inlet port toward the guide groove 320. The barrier structure 331 could prevent liquid from flowing backward from the guide groove 320 toward the inlet port by using the difference in cross-sectional area of the fluid path. This is because fluid resistance is greater in areas with a smaller cross-sectional area than in areas with a large cross-sectional area. Here, the direction of from the inlet port toward the guide groove 320 refer to the liquid flow direction, and the cross-sectional area may be calculated as the product of the width of the floor surface and the height of the side wall of the guide groove 320.

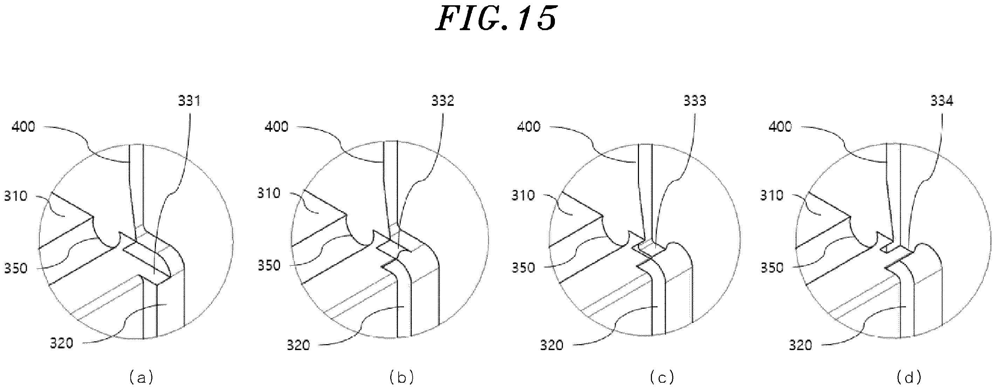

FIG. 15 is a partially enlarged view illustrating various embodiments of a barrier structure.

Referring to (a) and (b) of FIG. 15, the barrier structures 331 and 332 may include a step in the depth direction that increases in depth from the inlet port toward the guide groove 320. The barrier structures 331 and 332 may be located at or adjacent to the upper wall of the accommodation room 310 and may include a step so that the depth on the opposite side is greater than on the inlet port side. The barrier structures 331 and 332 could prevent liquid from flowing back from the guide groove 320 toward the inlet port by using the difference in depth of the fluid path.

Specifically, as shown in (a) of FIG. 15, the barrier structure 331 may include the step in the depth direction where the depth increases at a portion connection the guide groove 320 formed on the upper wall of the accommodation room 310 to the guide groove 320 formed on the side wall of the accommodation room 310. Alternatively, as shown in (b) of FIG. 15, the barrier structure 332 may include the step in the depth direction where the depth increases in the guide groove 320 formed on the upper wall of the accommodation room 310 adjacent to the inlet port.

Referring to (c) and (d) of FIG. 15, the barrier structures 333 and 334 may include an obstacle wall that separates the inlet port and the guide groove 320. The obstacle walls 333 and 334 may be part of the upper wall of the accommodation room 310 or may include a micro-channel formed on the upper wall.

The guide groove 320 may be provided at a certain distance from the inlet port. Obstacle walls 333 and 334, which are part of the accommodation room 310, may be provided between the inlet port and the guide groove 320. The obstacle walls 333 and 334 may increase fluid resistance by providing a portion in the middle of the channel where the channel is suddenly narrowed or cut off, thereby preventing the liquid from flowing back from the guide groove 320 toward the inlet port.

The distance between the inlet port and the guide groove 320 or the width of the obstacle wall 333 and 334 may be set to be smaller than the radius of the maximum size of the liquid droplet flowing in through the inlet port. The liquid flowing in through the inlet port condenses into droplet at the outlet of the inlet port. As the droplet grows larger and comes into contact with the guide groove 320, a flow of liquid is formed that is sucked toward the guide groove 320 by one or more of capillary force, cohesive force, and adhesion force.

Specifically, as shown in (c) of FIG. 15, the obstacle wall 333 may be located between the inlet port and the guide groove 320 and may include a micro-channel formed on the upper wall of the accommodation room 310. Alternatively, as shown in (d) of FIG. 15, the obstacle wall 334 may be located between the inlet port and the guide groove 320 and may be provided as part of the upper wall of the accommodation room 310.

The chamber 300 may further include a blocking protrusion 350 provided on the upper wall of the accommodation room 310, and the blocking protrusion 350 may be provided on the opposite side of inlet port where the guide groove 320 is located with respect to the inlet port. The blocking protrusion 350 may block the liquid discharged through the inlet port from flowing to the opposite direction where the guide groove 320 is not provided.

The inlet port and the blocking protrusion 350 may be disposed apart from each other. At this time, the distance between the inlet port and the blocking protrusion 350 may be set to be smaller than the radius of the maximum size of the liquid droplet flowing in through the inlet port. The degree of protrusion of the blocking protrusion 350 may be set to be greater than the radius of the maximum size of the liquid droplet flowing in through the inlet port. The liquid flowing in through the inlet port condenses into droplet at the outlet of the inlet port. As the droplets grow larger and come into contact with the blocking protrusion 350, the flow of liquid in the opposite direction of the guide groove 320 is blocked.

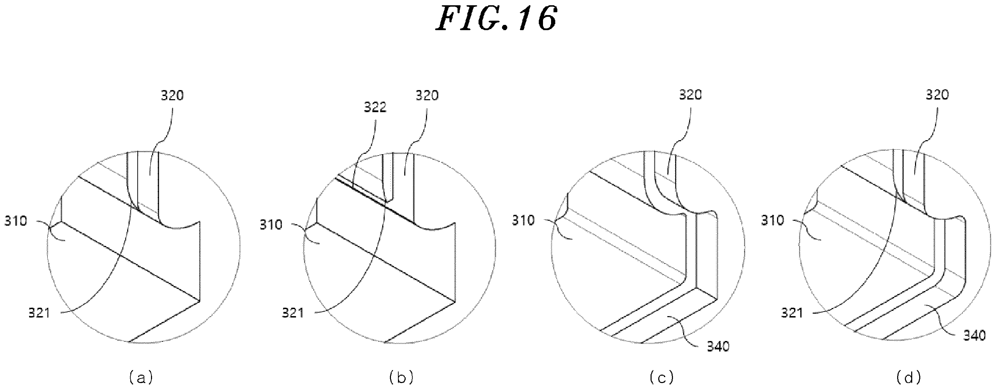

FIG. 16 is a partially enlarged view illustrating various embodiments of a lower portion of the waste chamber.

Referring to FIG. 16, the guide groove 320 may include a portion 321 whose cross-sectional area becomes smaller as it goes downward in the direction of gravity of the accommodation room 310. For example, the width of the bottom surface of the guide groove 320 may decrease as it moves downward in the direction of gravity of the accommodation room 310.

In addition, the side wall of the accommodation room 310 may include a shape extending outward from the end of the portion 321 where the width of the bottom surface of the guide groove 320 is reduced. Here, the outward refers to the width direction (perpendicular to the direction of gravity) of the accommodation room 310. At this time, as shown in (a) of FIG. 16, the guide groove 320 may be terminated with the width of the bottom surface reduced at a portion 321 where the side wall of the accommodation room 310 extends outward. Alternatively, as shown in (c) of FIG. 16, the guide groove 320 may extend to the same width on the bottom surface even in the portion where the side wall of the accommodation room 310 extends outward.

The liquid flowing along the guide groove 320 may fall in the direction of gravity when the width of the bottom surface of the guide groove 320 decreases and the side wall of the accommodation room 310 expands outward at the point where the guide groove 320 ends. That is, the liquid flowing along the guide groove 320 may fall toward the lower wall of the accommodation room 310.

Referring to (b) of FIG. 16, the chamber 300 may further include a floor connection groove 322 that connects the guide groove 320 to the lower wall or floor of the accommodation room 310. The liquid flowing along the guide groove 320 in the direction of gravity may flow to the lower wall or floor of the accommodation room 310 along the floor connection groove 322. The liquid fallen on the floor of the accommodation room 310 may flow toward the lower wall of the accommodation room 310.

Referring to (c) and (d) of FIG. 16, the chamber 300 may further include a capillary groove 340 provided on the lower wall of the accommodation room 310. The capillary groove 340 may have a cross-sectional area within a range where capillary action of the liquid flowing in through the inflow channel 400 could occur. The capillary groove 340 may extend to the side wall of the accommodation room 310 where the guide groove 320 is formed. Referring to (c) of FIG. 16, the guide groove 320 may be connected to the capillary groove 340.

The above-described embodiments are merely examples, and it will be appreciated by one of ordinary skills in the art various changes may be made thereto without departing from the scope of the disclosure. Accordingly, the embodiments set forth herein are provided for illustrative purposes, but not to limit the scope of the disclosure, and should be appreciated that the scope of the disclosure is not limited by the embodiments. The scope of the disclosure should be construed by the following claims, and all technical spirits within equivalents thereof should be interpreted to belong to the scope of the disclosure.

### [Legend of reference numbers]

10: target analyte detection cartridge
11: base,
12: liquid storage chamber connecting portion,
101: sample chamber, 102: metering chamber,
103: waste chamber,
104a: first mixing chamber, 104b: second mixing chamber,
105: detection chamber, 106: detection buffer chamber,
107: shuttle chamber, 108: ante chamber,
111: first pneumatic channel, 112: first pneumatic port,
113: second pneumatic channel, 114: second pneumatic port,
121: sample channel, 122: metering channel,
123: mixing channel, 124: ante channel,
125: first waste channel, 126: second waste channel,
127: mixing connection channel, 128: shuttle channel,
131: first buffer channel, 132: a second buffer channel,
133: third buffer channel,
135: detection channel,
141-145: first to fifth valves,
161: lysis buffer chamber, 162: binding buffer chamber,
163,164: washing buffer chamber, 165: elution buffer chamber,
300: chamber, 310: accommodation room,
320: guide groove, 330: barrier structure,
340: capillary groove, 350: blocking protrusion,
400: inflow channel.

## Claims

1. A cartridge (10) for detecting a target analyte, comprising:
a base (11) comprising a first surface, a second surface opposite to the first surface, and side surfaces connecting between the first surface and the second surface;
a chamber (300) recessed from the first surface of the base; and
an inflow channel (400) recessed from the first surface of the base and connected to upper portion of the chamber,
wherein the chamber comprises an accommodation room (310) for accommodating liquid, an inlet port connected the inflow channel, a guide groove (320) for guiding liquid introduced from the inlet port downward to the accommodation room, and a barrier structure (330) for preventing back flow of liquid from the guide groove toward the inlet port.

2. The cartridge according to claim 1,
wherein the accommodation room comprises a concave surface, an upper wall provided the inlet port, a lower wall, and side walls connecting between the upper wall and the lower wall, and
wherein the guide groove comprises a portion extending downward in the direction of gravity from the side wall of the accommodation room.

3. The cartridge according to claim 2,
wherein the inlet port is located away from the side wall of the accommodation room,
wherein the guide groove comprises a first portion extending along the upper wall of the accommodation room to adjacent the side wall and a second portion extending downward in the direction of gravity along the side wall of the accommodation room.

4. The cartridge according to claim 1,
wherein the guide groove comprises a portion which is deeper than the depth of the inlet port and shallower than the depth of the accommodation room,
wherein preferably the guide groove is configured to have a cross-sectional area within a range where a capillary phenomenon of the liquid introduced through the inflow channel could occur.

5. The cartridge according to claim 2,
wherein the barrier structure comprises a step that deepens in the direction from the inlet port toward the guide groove, or
wherein the barrier structure comprises a step that increases the cross-sectional area in the direction from the inlet port toward the guide groove.

6. The cartridge according to claim 2,
wherein the barrier structure comprises a barrier wall separating the inlet port and the guide groove,
wherein preferably the barrier wall is provided with a part of the upper wall of the accommodation room.

7. The cartridge according to claim 6,
wherein a distance between the inlet port and the guide groove is provided smaller than the maximum diameter of the droplet of the liquid introduced through the inflow channel.

8. The cartridge according to claim 2,
wherein the chamber further comprises a capillary groove (340) provided on the lower wall of the accommodation room, and
wherein the capillary groove is configured to have a cross-sectional area within a range where a capillary phenomenon of the liquid introduced through the inflow channel could occur.

9. The cartridge according to claim 8,
wherein the capillary groove extends to the side wall of the accommodation room where the guide groove is provided,
wherein preferably the guide groove is connected to the capillary groove.

10. The cartridge according to claim 2,
wherein the guide groove comprises a portion in which a cross-sectional area becomes smaller downward in the direction of gravity of the accommodation room.

11. The cartridge according to claim 1,
wherein the chamber further comprises a floor connection groove connecting the guide groove to a floor of the accommodation room.

12. The cartridge according to claim 1,
wherein the chamber further includes a blocking protrusion (350) provided on the upper wall of the accommodation room, and
wherein the blocking protrusion is provided in a direction opposite to the guide groove based on the inlet port,
wherein preferably the inlet port and the blocking protrusion are disposed apart from each other.

13. The cartridge according to claim 1,
further comprising a cover provided on the one surface of the base to seal the inflow channel and the chamber.

14. The cartridge according to claim 1,
further comprising:
a sample chamber into which a sample is introduced;
a metering chamber connected to the sample chamber for quantifying the sample;
a mixing chamber connected to the metering chamber and accommodating a magnetic bead;
a liquid storage chamber connected to the mixing chamber and storing liquid;
a detection chamber for detecting the target analyte;
a waste chamber connected to the metering chamber;
a plurality of liquid transport channels for transporting liquid of the liquid storage chamber to the mixing chamber; and
a waste channel connecting the metering chamber and the waste chamber,
wherein the chamber comprises the waste chamber, and the inflow channel comprises the waste channel.

15. The cartridge according to claim 14,
further comprising a drain channel connecting the mixing chamber and the waste chamber,
wherein the inflow channel comprises the drain channel.
